# EUROPÄISCHE PATENTSCHRIFT

(11) **EP 2 866 935 B1**
(45) Veröffentlichungstag und Bekanntmachung des Hinweises auf die Patenterteilung: **10.10.2018**
(21) Anmeldenummer: 13733269.8
(22) Anmeldetag: 26.06.2013
(51) Int. Cl.: B01J 29/40, B01J 29/70, B01J 29/80, B01J 37/02, C07C 1/20

(54) **VERFAHREN FÜR DIE UMWANDLUNG VON OXYGENATEN ZU OLEFINEN**
PROCESS FOR THE CONVERSION OF OXYGENATES TO OLEFINS
PROCÉDÉ DE CONVERSION D'HYDROCARBURES OXYGÉNÉS EN OLEFINES

(30) Priorität: 29.06.2012 EP 12174295
(43) Veröffentlichungstag der Anmeldung: 06.05.2015
(73) Patentinhaber: BASF SE, 67056 Ludwigshafen am Rhein (DE)
(72) Erfinder: SPANNHOFF, Kirsten, 67063 Ludwigshafen (DE); PATCAS, Florina, Corina, 67065 Ludwigshafen (DE); SCHWAB, Ekkehard, 67434 Neustadt (DE); WECK, Alexander, 67251 Freinsheim (DE); BAY, Kerem, 67063 Ludwigshafen (DE)
(86) Internationale Anmeldenummer: PCT/EP2013/063435
(87) Internationale Veröffentlichungsnummer: WO 2014/001411

(56) Entgegenhaltungen:
- EP-A1- 2 446 964
- JP-A- 2007 137 840
- US-A1- 2002 038 775
- PATCAS, F. C.: JOURNAL OF CATALYSIS, Bd. 231, 2005, Seiten 194-200, XP002717371, in der Anmeldung erwähnt
- ANTIA ET AL.: IND. ENG. CHEM. RES., Bd. 34, 1995, Seiten 140-147, XP002717372, in der Anmeldung erwähnt
- LEE ET AL.: CATAL. LETT, Bd. 129, 2009, Seiten 408-415, XP002717373, in der Anmeldung erwähnt
- HAMMON ET AL.: APPLIED CATALYSIS, Bd. 37, 1988, Seiten 155-174, XP002717374, in der Anmeldung erwähnt

## Beschreibung

Die vorliegende Erfindung betrifft ein Verfahren zur Umwandlung von Ethern zu Olefinen, unter Verwendung eines Katalysators in Form eines beschichteten Trägersubstrats sowie unter Verwendung eines Katalysators zur Umwandlung von Ethern zu Olefinen welcher gemäß vorliegender Erfindung erhältlich ist.

### EINLEITUNG

Angesichts zunehmender Knappheit von Erdölvorkommen, welche als Ausgangsmaterial zur Herstellung von niederen Kohlenwasserstoffen und deren Derivaten dienen, gewinnen alternative Verfahren zur Herstellung solcher Grundchemikalien zunehmend an Bedeutung. In alternativen Verfahren zur Erzeugung von niederen Kohlenwasserstoffen und deren Derivaten werden häufig spezifische Katalysatoren eingesetzt, um aus anderen Rohstoffen und/oder Chemikalien niedere Kohlenwasserstoffe und deren Derivate wie insbesondere ungesättigte niedere Kohlenwasserstoffe möglichst selektiv zu erzeugen. Hierbei spielen Verfahren eine große Rolle, in welchen Methanol als Ausgangschemikalie einer katalytischen Umwandlung unterzogen wird, wodurch in der Regel ein Gemisch aus Olefinen, Paraffinen und Aromaten entsteht.

Bei solchen katalytischen Umwandlungen besteht die Herausforderung insbesondere darin, die darin verwendeten Katalysatoren sowie die Prozessführung und deren Parameter in einer Weise zu verfeinern, dass möglichst gezielt einige wenige Produkte möglichst selektiv in der katalytischen Umwandlung entstehen. Somit werden diese Verfahren insbesondere nach den Produkten benannt, welche hauptsächlich erzeugt werden. In den vergangenen Jahrzehnten haben solche Verfahren eine besondere Bedeutung erlangt, welche die Umwandlung von Methanol zu Olefinen ermöglichen und entsprechend als Methanol-zu-Olefin-Verfahren (MTO-Verfahren für methanol to olefins) gekennzeichnet werden. Hierzu wurden insbesondere Katalysatoren und Verfahren entwickelt, welche Methanol über das Zwischenprodukt Dimethylether zu Gemischen umwandeln, deren Hauptbestandteile Ethen und Propen ausmachen.

Antia et al. in Ind. Eng. Chem. Res. 1995, 34, Seiten 140-147 beschreibt die Beschichtung eines Trägersubstrats mit ZSM-5 und dessen Verwendung in einem Methanol-zu-Benzin-Verfahren (MTG-Verfahren).

US 4,692,423 betrifft ein Verfahren zur Herstellung eines geträgerten zeolithhaltigen Katalysators durch Auftragung eines Gemischs eines Zeoliths in einem polymerisierbaren Lösungsmittel wie beispielsweise Tetrahydrofuran auf ein poröses Trägersubstrat, wobei letzteres aus organischem oder anorganischem Material bestehen kann.

Ivanova et al. in J. Phys. Chem. C 2007, 111, Seiten 4368-4374 betrifft einen geschäumten Formkörper sowie ein Extrudat aus β-Siliciumcarbid, auf welche jeweils eine ZSM-5-Beschichtung aufgetragen wird, sowie die Verwendung eines solchen beschichten Schaumkörpers und Extrudats in Methanol-zu-Olefin-Verfahren (MTO-Verfahren). Im Vergleich zur Verwendung des pulverförmigen Zeolithen an sich wird hierbei eine Verbesserung der katalytischen Aktivität/Selektivität beobachtet, wobei die beschichteten Katalysatoren eine höhere Stabilität gegenüber einer Deaktivierung durch Verkokung zeigen sollen.

Patcas, F. C. in Journal of Catalysis 2005, 231, Seiten 194-200 beschreibt mit ZSM-5-Zeolith beschichtete keramische Schäume sowie deren Verwendung in Methanol-zu-Olefin-Verfahren. Insbesondere wird beschrieben, dass im Vergleich zu zeolithhaltigen Pellets solche beschichteten keramischen Schäume eine Verbesserung der Aktivität und Selektivität zeigen sollen. Bei niedrigeren Temperaturen und höheren Raumgeschwindigkeiten werden allerdings im Vergleich zu den zeolithhaltigen Pellets geringere Raumausbeuten beschrieben.

WO 98/29519 A1 beschreibt auf anorganischen Materialien geträgerte nichtzeolithische Molsiebe und insbesondere SAPO sowie deren Verwendung in Methanol-zu-Olefin-Verfahren.

WO 94/25151 A1 beschreibt auf Monolithen geträgerte Zeolithe und insbesondere ZSM-5 sowie deren Verwendung als Molsieb in Trennungsverfahren.

Hammon et al. in Applied Catalysis 1988, 37, Seiten 155-174 betrifft Verfahren zur Herstellung von Zeolithextrudaten mit wenig bis keinem Bindemittel und deren Verwendung in Methanol-zu-Olefin-Verfahren. Allerdings wird in Hammon et al. die Verwendung von zu Monolithen geformter Extrudate als Katalysatoren wegen rascher Verkokung und damit entsprechend verbundener geringer Standzeiten als besonders nachteilig beschrieben.

Li et al. in Catal. Lett. 2009, 129, Seiten 408-415 betrifft einen geschäumten ZSM-5-Monolithen sowie dessen Verwendung in einem Methanol-zu-Olefin-Verfahren.

US 4,049,573 betrifft ein katalytisches Verfahren zur Umwandlung von niederen Alkoholen und deren Ether und insbesondere Methanol und Dimethylether selektiv zu einem Kohlenwasserstoffgemisch mit hohem Anteil an C2-C3-Olefinen sowie einkernige Aromaten und insbesondere Para-Xylol.

Goryainova et al. in Petroleum Chemistry 2011, Bd. 51, Nr. 3, S. 169-173 beschreibt die katalytische Umwandlung von Dimethylether zu niederen Olefinen unter Verwendung von Magnesium-haltigen Zeolithen.

JP 2007 137840 betrifft ein Verfahren zur Umwandlung von Oxygenaten zu Xylol unter Verwendung eines Zeolithkatalysators, wobei der Zeolith als Schicht auf einem Träger, wie zum Beispiel Cordierit, vorliegen kann.

US 2002/038775 A1 offenbart ein Verfahren zur Umwandlung von Oxygenaten zu Olefinen in Gegenwart eines Katalysators, der beispielsweise einen Zeolithen des MFI-Strukturtyps umfasst, wobei Temperaturen zwischen 275 und 600 °C offenbart werden. Der verwendete Katalysator wird unter Verwendung eines Trägermaterials bestehend aus einem organischen Ionenaustauscher hergestellt, wobei der organische Ionenaustauscher nach der Herstellung entfernt wird.

EP 2 446 964 A1 betrifft einen Katalysator, welcher mittels Extrusion hergestellt wird, und der in einem Verfahren zur Umwandlung von Oxygenaten zu Olefinen eingesetzt wird.

Obwohl im Stand der Technik hinsichtlich der Selektivitäten und/oder Aktivitäten der Katalysatoren durch Änderungen ihrer Zusammensetzung und/oder ihrer Ausgestaltung insbesondere auch in Methanol-zu-Olefin-Verfahren teilweise Fortschritte erzielt werden konnten, besteht nach wie vor ein erheblicher Bedarf an neuen Katalysatoren und Verfahren, welche neben neuen und/oder verbesserten Selektivitäten auch eine bessere Resistenz gegenüber der allfälligen Deaktivierung in solchen Verfahren aufweisen. Dies gilt insbesondere für solche Verbesserungen, welche zu einer geringeren Verkokung des Katalysators führen können, um dadurch eine höhere Effizienz bestehender und neuer Prozesse ermöglichen zu können.

### DETAILLIERTE BESCHREIBUNG

Somit bestand die Aufgabe der vorliegenden Erfindung darin, einen verbesserten Katalysator bereitzustellen, insbesondere für die Umwandlung von Oxygenaten zu Olefinen, welcher eine höhere Standzeit des Katalysators bei vergleichbarer Raumgeschwindigkeit und Umsatz an Oxygenaten ermöglicht. Hierbei bestand die Aufgabe der vorliegenden Erfindung insbesondere darin, Verbesserungen hinsichtlich der Verkokung des Katalysators, welche beispielsweise in Methanol-zu-Olefin-Verfahren die Standzeiten eines Katalysators bedingt, bis zu welchen die Regenerierung des Katalysators erforderlich ist, zu bewirken, um die gewünschte Selektivität und/oder eine ausreichende Raumzeitausbeute zu erzielen.

Es wurde überraschenderweise herausgefunden, dass durch die kombinierte Verwendung eines ein oder mehreren Ethern enthaltenden Gasstroms mit einem Katalysator welcher ein Trägersubstrat und eine auf das Substrat aufgebrachte Schicht aufweist, wobei die katalytisch aktive Schicht ein oder mehrere Zeolithe vom MFI-Strukturtyp, ein Verfahren zur Herstellung von Olefinen bereitgestellt werden kann, welches erheblich längere Standzeiten des Katalysators ermöglicht. Insbesondere wurde unerwarteterweise herausgefunden, dass in einem Verfahren zur Herstellung von Olefinen eine unerwartete Verbesserung der Resistenz des Katalysators gegenüber einer Deaktivierung während dessen Verwendung realisiert werden kann bei Verwendung eines solchen beschichteten Trägersubstrats als Katalysator, wenn der Eduktstrom ein oder mehrere Ether enthält.
Somit betrifft die vorliegende Erfindung Verfahren zur Umwandlung von Ethern zu Olefinen umfassend
(1) Bereitstellen eines Gasstroms enthaltend ein oder mehrere Ether;
(2) Inkontaktbringen des in (1) bereitgestellten Gasstroms mit einem Katalysator, wobei das Inkontaktbringen bei einer Temperatur im Bereich von 430 bis520°C erfolgt;
(3) Kalzinierung des Katalysators zur Regenerierung wobei die Kalzinierung bei einer Temperatur im Bereich von 450 bis 550°C in Luftatmosphäre erfolgt;
(4) Bereitstellen eines Gasstroms enthaltend ein oder mehrere Ether;
(5) Inkontaktbringen des in (4) bereitgestellten Gasstroms mit dem regenerierten Katalysator,
   wobei der Katalysator
   - ein Trägersubstrat und
   - eine auf das Substrat aufgebrachte Schicht umfasst,
   wobei die Schicht ein oder mehrere Zeolithe vom MFI -Strukturtyp enthält, und wobei das Trägersubstrat keramische und/oder metallische Stoffe enthält.

Somit wird herein ein Verfahren zur Umwandlung von Ethern zu Olefinen umfassend offenbart
(1) Bereitstellen eines Gasstroms enthaltend ein oder mehrere Ether;
(2) Inkontaktbringen des in (1) bereitgestellten Gasstroms mit einem Katalysator; wobei der Katalysator
   - ein Trägersubstrat und
   - eine auf das Substrat aufgebrachte Schicht umfasst,
   wobei die Schicht ein oder mehrere Zeolithe vom MFI, MEL und/oder vom MWW-Strukturtyp enthält

Bezüglich des im erfindungsgemäßen Verfahren verwendeten Trägersubstrats besteht prinzipiell keinerlei Einschränkung hinsichtlich dessen Form. Somit kann im Prinzip jede denkbar mögliche Form für das Trägersubstrat gewählt werden, vorausgesetzt, diese ist geeignet, um mit einer Schicht des einen oder der mehreren Zeolithe vom MFI-Strukturtyp zumindest teilweise überzogen zu werden. Gemäß vorliegender Erfindung ist es jedoch bevorzugt, dass die Form des Trägersubstrats aus der Gruppe bestehend aus Granulaten, Pellets, Netzen, Ringen, Kugeln, Zylindern, Hohlzylindern, Monolithen und Mischungen und/oder Kombinationen von zwei oder mehr davon ausgewählt ist. Hinsichtlich der bevorzugten Mischungen beziehen sich diese bevorzugt auf solche Formen des Trägersubstrats, welche zur Herstellung von Schüttungen gewöhnlich verwendet werden, wobei dies insbesondere die bevorzugten Formen des Trägersubstrats ausgewählt aus der Gruppe der Granulate, Pellets, Netze, Ringe, Kugeln, Zylinder und Hohlzylinder betrifft. Andererseits, hinsichtlich der Kombinationen von Formen des Trägersubstrats gemäß vorliegender Erfindung werden solche Kombinationen aus Schüttungen und Monolithen bevorzugt, wobei die Schüttungen bevorzugt Trägersubstrate ausgewählt aus der Gruppe bestehend aus Granulaten, Pellets, Netzen, Ringen, Kugeln, Zylindern, Hohlzylindern und Mischungen von zwei oder mehr davon enthalten. Insbesondere betreffen solche Kombinationen aus Schüttungen und Monolithen bevorzugte Formen des Katalysators, in welchen eine Abfolge von einem oder mehreren Monolithen und einer oder mehreren Schüttungen enthalten ist, bei welchen die Schüttung(en) und Monolith(en) einzelne Zonen des Katalysators bilden. Alternativ werden jedoch auch Ausführungsformen des erfindungsgemäßen Katalysators bevorzugt, welche Kombinationen von Monolithen als Form des Trägersubstrats enthalten, insbesondere Kombinationen von Monolithen gemäß den besonderen oder bevorzugten Ausführungsformen wie in der vorliegenden Anmeldung beschrieben. Gemäß besonders bevorzugten Ausführungsformen der vorliegenden Erfindung besteht das Trägersubstrat aus einem oder mehreren Monolithen, wobei bei der Verwendung mehrerer Monolithe bevorzugt eine Abfolge und/oder eine Aneinanderreihung von einzelnen oder mehreren mindestens paarweise nebeneinander angeordneten Monolithen in dem Katalysator enthalten ist.

Somit werden gemäß der vorliegenden Erfindung Ausführungsformen des Verfahrens zur Umwandlung von Ethern zu Olefinen bevorzugt, bei welchen die Form des Trägersubstrats ausgewählt ist aus der Gruppe bestehend aus Granulaten, Pellets, Netzen, Ringen, Kugeln, Zylinder, Hohlzylinder, Monolithen und Mischungen und/oder Kombinationen von zwei oder mehr davon, wobei das Trägersubstrat bevorzugt ein oder mehrere Monolithe ist.

Bezüglich des einen oder der mehreren Monoliths, welche bevorzugt als Trägersubstrat im Katalysator des erfindungsgemäßen Verfahren verwendet werden, besteht wiederum prinzipiell keine Einschränkung hinsichtlich der Form, welche der eine oder die mehreren Monolithe annehmen können. Gemäß vorliegender Erfindung werden Monolithe bevorzugt, welche ausgewählt sind aus der Gruppe bestehend aus Waben, Geflechten, Schäumen und Kombinationen von zwei oder mehr davon, wobei weiter bevorzugt der eine oder die mehreren Monolithe ein oder mehrere Waben und/oder Geflechte enthalten. Besonders bevorzugt gemäß vorliegender Erfindung haben der eine oder die mehreren Monolithe, welche bevorzugt als Trägersubstrat verwendet werden, Wabenform.

Somit werden gemäß der vorliegenden Erfindung Ausführungsformen des Verfahrens zur Umwandlung von Ethern zu Olefinen bevorzugt, bei welchen der ein oder die mehreren Monolithe als bevorzugter Trägersubstrat ausgewählt sind aus der Gruppe bestehend aus Waben, Geflechte, Schäume, und Kombinationen von zwei oder mehr davon, wobei der eine oder die mehreren Monolithe bevorzugt Wabenform haben.

Gemäß den bevorzugten Ausführungsformen des Verfahrens, in welchen der Katalysator einen oder mehrere Monolithe mit Wabenform enthält, bestehen keinerlei besondere Einschränkungen hinsichtlich der Wabenform, vorausgesetzt, diese ist dazu geeignet, zumindest teilweise mit dem einen oder den mehreren Zeolithen vom MFI-Strukturtyp beschichtet zu werden. Nach besonders bevorzugten Ausführungsformen besteht die Wabe aus einer Vielzahl parallel zueinander laufender Kanäle, welche durch die Wände des Monoliths voneinander getrennt sind, wobei bevorzugt die Form der Kanäle und/oder bevorzugt die Dicke der Wände des Monoliths, welche die Kanäle voneinander trennen bis auf eine gewisse Toleranz sowohl bezüglich der Form der Kanäle als auch hinsichtlich der Wanddicke, welche durch den zur Herstellung des Monoliths verwendeten Stoff bzw. durch die Herstellungsweise der Wabe bzw. der Wabenform üblicherweise gegeben ist, gleich sind. So werden beispielsweise Kanäle bevorzugt, welche eine eckige Form aufweisen, bevorzugt die Form eines regelmäßigen Polyeders mit drei oder mehr Ecken, bevorzugt mit drei, vier oder sechs Ecken und besonders bevorzugt mit vier Ecken. Hinsichtlich der Dimensionen der Kanäle in den bevorzugten Ausführungsformen der Monolithe mit Wabenform besteht prinzipiell keine Einschränkung, sofern die gewählten Dimensionen eine zumindest teilweise Beschichtung des Monoliths mit Wabenform als Trägersubstrat im erfindungsgemäßen Katalysator mit dem einen oder den mehreren Zeolithen vom MFI-Strukturtyp erlaubt. Somit können gemäß vorliegender Erfindung beispielsweise Monolithe mit Wabenform verwendet werden, welche 62 bis 186 Kanäle pro Quadratzentimeter (400 bis 1.200 cpsi = cells per square inch) aufweisen, wobei Monolithe mit Wabenform bevorzugt werden mit 78 bis 171 Kanäle pro Quadratzentimeter (500 bis 1.100 cpsi), weiter bevorzugt solche mit 93 bis 163 (600 bis 1.050 cpsi), weiter bevorzugt solche mit 109 bis 155 (700 bis 1.000 cpsi), weiter bevorzugt solche mit 124 bis 147 (800 bis 950 cpsi) und weiter bevorzugt solche mit 132 bis 144 (850 bis 930 cpsi). Gemäß besonders bevorzugten Ausführungsformen der vorliegenden Erfindung nach welchen das Trägersubstrat ein oder mehrere Monolithe mit Wabenform enthält, werden solche mit 136 bis 141 Kanäle pro Quadratzentimeter (880 bis 910 cpsi) verwendet.

Gemäß alternativen Ausführungsformen der vorliegenden Erfindung, welche einen oder mehrere Monolithe als Trägersubstrat im Katalysator verwenden, sind hierbei keine schaumförmigen Substrate darin enthalten. Somit werden Ausführungsformen des im Verfahren verwendeten Katalysators gleichfalls bevorzugt, in welchen das Trägersubstrat keine Schäume und insbesondere keine Schäume als Monolith enthält.

Was den Stoff betrifft, aus welchem das Trägersubstrat besteht, und insbesondere die darin enthaltenen Schüttungen und/oder Monolithe, bestehen gemäß vorliegender Erfindung diesbezüglich keinerlei Einschränkungen, vorausgesetzt er ist dazu geeignet, mit dem einen oder den mehreren Zeolithen vom MFI-Strukturtyp zumindest teilweise beschichtet zu werden. Somit kann im Prinzip jedes geeignete Material und/oder jeder Materialverbund als Stoff für das Trägersubstrat verwendet werden, wobei bevorzugt solche Materialien verwendet werden, welche eine hohe Temperaturbeständigkeit aufweisen und/oder in hohem Maß hinsichtlich ihrer chemischen Reaktivität inert sind. Somit werden bevorzugt keramische und/oder metallische Stoffe sowie Verbundmaterialien keramischer und/oder metallischer Stoffe als Trägersubstrat im erfindungsgemäßen Katalysator verwendet, wobei bevorzugt keramische Stoffe als Trägersubstrat Verwendung finden. Hinsichtlich der bevorzugten keramischen Stoffe werden bevorzugt ein oder mehrere dieser Stoffe ausgewählt aus der Gruppe bestehend aus Aluminiumoxid, Siliziumoxid, Silikaten, Aluminosilikaten, Siliziumcarbid, Cordierit, Mullit, Zirkon, Spinellen, Magnesiumoxid, Titandioxid und Mischungen aus zwei oder mehr davon. Gemäß besonders bevorzugten Ausführungsformen vorliegender Erfindung werden die für das Trägersubstrat bevorzugt verwendeten keramischen Stoffe ausgewählt aus der Gruppe bestehend aus α-Aluminiumoxid, Siliziumkarbid, Cordierit und Mischungen aus zwei oder mehr davon. Gemäß besonders bevorzugten Ausführungsformen enthält das Trägersubstrat Cordierit, wobei weiter bevorzugt das Trägersubstrat ein Cordierit-Substrat ist.

Somit werden gemäß der vorliegenden Erfindung Ausführungsformen des Verfahrens zur Umwandlung von Ethern zu Olefinen bevorzugt, bei welchen das Trägersubstrat keramische und/oder metallische Stoffe, bevorzugt keramische Stoffe, weiter bevorzugt ein oder mehrere Stoffe ausgewählt aus der Gruppe bestehend aus Aluminiumoxid, Siliziumoxid, Silikaten, Aluminosilikaten, Siliziumcarbid, Cordierit, Mullit, Zirkon, Spinellen, Magnesiumoxid, Titandioxid und Mischungen aus zwei oder mehr davon, bevorzugt aus der Gruppe bestehend aus alpha-Aluminiumoxid, Siliziumcarbid, Cordierit und Mischungen aus zwei oder mehr davon, enthält, wobei das Trägersubstrat besonders bevorzugt ein Cordierit-Substrat ist.

Bezüglich der einen oder mehreren Zeolithe, welche in dem Katalysator enthalten sind, bestehen gemäß vorliegender Erfindung keinerlei Einschränkungen weder hinsichtlich der Art noch bezüglich der Anzahl an Zeolithen, welche hierin Verwendung finden können, vorausgesetzt es handelt sich dabei um Zeolithe vom Strukturtyp MFI.

Gemäß der vorliegenden Erfindung werden Zeolithe vom MFI-Strukturtyp im Katalysator des erfindungsgemäßen Verfahrens zur Umwandlung von Ethern zu Olefinen verwendet. Diesbezüglich besteht keinerlei Einschränkung hinsichtlich Art und/oder Zahl der verwendeten Zeolithe diesen Strukturtyps, wobei der eine oder die mehreren Zeolithe vom MFI-Strukturtyp, welche im erfindungsgemäßen Katalysator verwendet werden, bevorzugt ausgewählt sind aus der Gruppe bestehend aus ZSM-5, ZBM-10, [As-Si-O]-MFI, [Fe-Si-O]-MFI, [Ga-Si-O]-MFI, AMS-1B, AZ-1, Bor-C, Boralit C, Encilit, FZ-1, LZ-105, monoklinem H-ZSM-5, Mutinait, NU-4, NU-5, Silikalit, TS-1, TSZ, TSZ-III, TZ-01, USC-4, USI-108, ZBH, ZKQ-1B, ZMQ-TB und Mischungen aus zwei oder mehr davon. Weiter bevorzugt gemäß vorliegender Erfindung enthält der Katalysator ZSM-5 und/oder ZBM-10 als Zeolith vom MFI-Strukturtyp, wobei besonders bevorzugt ZSM-5 als Zeolith verwendet wird. Bezüglich des zeolithischen Materials ZBM-10 und dessen Herstellung wird beispielsweise auf die EP 0 007 081 A1 sowie auf die EP 0 034 727 A2 verwiesen, deren Inhalt insbesondere hinsichtlich der Herstellung und Charakterisierung des Materials in die vorliegende Erfindung hiermit einbezogen wird.

Somit werden gemäß der vorliegenden Erfindung Ausführungsformen des Verfahrens zur Umwandlung von Ethern zu Olefinen bevorzugt, bei welchen der eine oder die mehreren Zeolithe vom MFI-Strukturtyp sind, und ausgewählt sind aus der Gruppe bestehend aus ZSM-5, ZBM-10, [As-Si-O]-MFI, [Fe-Si-O]-MFI, [Ga-Si-O]-MFI, AMS-1B, AZ-1, Bor-C, Boralit C, Encilit, FZ-1, LZ-105, monoklinem H-ZSM-5, Mutinait, NU-4, NU-5, Silikalit, TS-1, TSZ, TSZ-III, TZ-01, USC-4, USI-108, ZBH, ZKQ-1B, ZMQ-TB und Mischungen aus zwei oder mehr davon, weiter bevorzugt aus der Gruppe bestehend aus ZSM-5, ZBM-10, und Mischungen davon, wobei der Zeolith vom MFI-Strukturtyp bevorzugt ZSM-5 ist.

Gemäß einer bevorzugten Ausführungsform der vorliegenden Erfindung enthält der Katalysator keine wesentlichen Mengen eines oder mehrerer nicht-zeolithischer Materialien und insbesondere keine wesentlichen Mengen eines oder mehrerer Aluminosilikophosphate (SAPO). Im Sinne der vorliegenden Erfindung ist der Katalysator im Wesentlichen frei von bzw. enthält keine wesentlichen Mengen eines spezifischen Materials in Fällen, in denen dieses spezifische Material in einer Menge von 0,1 Gew.-% oder weniger im Katalysator vorhanden ist bezogen auf 100 Gew.-% der Gesamtmenge and dem einen oder den mehreren Zeolithen vom MFI-Strukturtyp, bevorzugt in einer Menge von 0,05 Gew.-% oder weniger, weiter bevorzugt von 0,001 Gew.-% oder weniger, weiter bevorzugt von 0,0005 Gew.-% oder weniger und weiter bevorzugt in einer Menge von 0,0001 Gew.-% oder weniger. Ein spezifisches Material im Sinne der vorliegenden Erfindung kennzeichnet insbesondere eine bestimmtes Element oder eine besondere Kombination an Elementen, ein bestimmter Stoff oder eine bestimmte Stoffmischung, sowie auch Kombinationen und/oder Mischungen von zwei oder mehr davon.

Zu den Aluminosilikophosphaten (SAPO) im Sinne der vorliegenden Erfindung zählen insbesondere die SAPO-Materialien SAPO-11, SAPO-47, SAPO-40, SAPO-43, SAPO-5, SAPO-31, SA-PO-34, SAPO-37, SAPO-35, SAPO-42, SAPO-56, SAPO-18, SAPO-41, SAPO-39 und CFSA-PO-1A.

Bezüglich der Form in welcher der eine oder die mehreren Zeolithe vom MFI-Strukturtyp im Katalysator des erfindungsgemäßen Verfahrens zur Umwandlung von Ethern zu Olefinen verwendet wird besteht prinizipiell keinerlei Einschränkung, insbesondere hinsichtlich der weiteren Elemente oder Verbindungen welche darin enthalten sein können. Somit besteht generell keinerlei Einschränkungen bezüglich der Ionen und Verbindungen welche in den Mikroporen des einen oder der mehreren Zeolithe enthalten sein können, insbesondere hinsichtlich der Gegenionen zum gegebenenfalls negativ geladenen Zeolithgerüst welche sich in den Mikroporen befinden. Entsprechend kann der eine oder die mehreren Zeolithe in einer Form vorliegen, in welcher die gegebenenfalls negative Ladung des Zeolithgerüsts durch eine oder mehrere verschiedene kationische Elemente und/oder Verbindungen kompensiert wird, wobei dies bevorzugt mindestens zum Teil durch ein oder mehrere kationische Elemente und/oder Verbindungen erfolgt welche ausgewählt sind aus der Gruppe bestehend aus H⁺, NH₄⁺, Li⁺, Na⁺, K⁺ und Kombinationen von zwei oder mehr davon, weiter bevorzugt aus der Gruppe bestehend aus H⁺, Na⁺, K⁺ und Kombinationen von zwei oder mehr davon. Gemäß besonders bevorzugten Ausführungsformen der vorliegenden Erfindung enthält der eine oder die mehreren Zeolithe vom MFI-Strukturtyp gegebenenfalls H⁺ und/oder Na⁺, und bevorzugt H⁺ als Gegenion zum negativ geladenen Zeolithgerüst, womit der eine oder die mehereren Zeolithe vom MFI-Strukturtyp besonders bevorzugt in ihrer jeweiligen H-Form im Katalysator des erfindungsgemäßen Verfahrens verwendet werden.

Bezüglich der Menge, in welcher das eine oder die mehreren Zeolithe vom MFI -Strukturtyp auf das Trägersubstrat im Katalysator gemäß der vorliegenden Erfindung aufgebracht sind, besteht prinzipiell keinerlei Einschränkung, vorausgesetzt, dass eine Schicht, welche den einen oder die mehreren Zeolithe enthält, zumindest teilweise auf dem Trägersubstrat gebildet werden kann. Somit weisen die erfindungsgemäßen Katalysatoren beispielsweise den einen oder die mehreren Zeolithe vom MFI-Strukturtyp auf in einer Gesamtbeladung von 0,005-1 g/cm³. Hierbei bezieht sich das Volumen auf das Volumen des beschichteten Trägersubstrats, wobei dies bei Hohlkörpern und/oder Aushöhlungen enthaltenden Körpern und Formen jene Hohlräume und Aushöhlungen mitumfasst. Nach einer alternativen Definition gemäß der vorliegenden Erfindung bezieht sich das Volumen bei der Beladung des Trägersubstrats bei Ausführungsformen, welche Schüttungen enthalten, auf das jeweilige Volumen der Schüttung einschließlich der darin enthaltenen Zwischen- und Hohlräume. Gemäß bevorzugten Ausführungsformen der vorliegenden Erfindung enthält der Katalysator den einen oder die mehreren Zeolithe vom MFI-Strukturtyp in einer Gesamtbeladung von 0,01-0,5 g/cm³ bezogen auf das Volumen des beschichteten Trägersubstrats und insbesondere auf dessen Volumen gemäß der zuvor genannten besonderen und bevorzugten Definitionen, weiter bevorzugt in einer Gesamtbeladung von 0,02-0,2 g/cm³, weiter bevorzugt von 0,04-0,1 g/cm³, weiter bevorzugt von 0,055-0,08 g/cm³ und weiter bevorzugt von 0,065-0,075 g/cm³. Gemäß besonders bevorzugten Ausführungsformen der vorliegenden Erfindung enthält der Katalysator den einen oder die mehreren Zeolithe vom MFI-Strukturtyp in einer Gesamtbeladung von 0,07-0,072 g/cm³ bezogen auf das Volumen des beschichteten Trägersubstrats gemäß den besonderen und bevorzugten Definitionen der vorliegenden Anmeldung.

Somit werden gemäß der vorliegenden Erfindung Ausführungsformen des Verfahrens zur Umwandlung von Ethern zu Olefinen bevorzugt, bei welchen der Katalysator den einen oder die mehreren Zeolithe vom MFI-Strukturtyp in einer Gesamtbeladung von 0,005 bis 1 g/cm³ enthält bezogen auf das Volumen des beschichteten Trägersubstrats, bevorzugt in einer Gesamtbeladung von 0,01 bis 0,5 g/cm³, weiter bevorzugt von 0,02 bis 0,2 g/cm³, weiter bevorzugt von 0,04 bis 0,1 g/cm³, weiter bevorzugt von 0,055 bis 0,08 g/cm³, weiter bevorzugt von 0,065 bis 0,075 g/cm³, und weiter bevorzugt in einer Gesamtbeladung von 0,07 bis 0,072 g/cm³.

Gleiches gilt entsprechend für der/die in dem Gasstrom gemäß (1) enthaltene(n) eine oder mehrere Ether, so dass gemäß dem erfindungsgemäßen Verfahren hierbei prinzipiell keinerlei Einschränkung gegeben ist, sofern der eine oder die mehreren Ether, welche in dem Gasstrom gemäß (1) enthalten sind, von einem der Katalysatoren gemäß vorliegender Erfindung und insbesondere gemäß ihren besonderen und bevorzugten Ausführungsformen zu mindestens einem Olefin beim Inkontaktbringen gemäß (2) umgewandelt werden können. Gemäß vorliegender Erfindung ist es jedoch bevorzugt, dass der eine oder die mehreren im Gasstrom gemäß (1) enthaltenen Ether ausgewählt sind aus der Gruppe bestehend aus Di-(C₁-C₃)alkylethern und Mischungen von zwei oder mehr davon. Weiter bevorzugt ist der eine oder die mehreren Ether ausgewählt aus der Gruppe bestehend aus Dimethylether, Diethylether, Ethylmethylether, Diisopropylether, Di-n-propylether und Mischungen von zwei oder mehr davon, wobei weiter bevorzugt der eine oder die mehreren Ether ausgewählt sind aus der Gruppe bestehend aus Dimethylether, Diethylether, Ethylmethylether und Mischungen von zwei oder mehr davon. Gemäß besonders bevorzugten Ausführungsformen des erfindungsgemäßen Verfahrens zur Umwandlung von Ethern zu Olefinen enthält der der Gasstrom gemäß (1) Dimethylether als der eine oder die mehreren Ether, wobei besonders bevorzugt Dimethylether der in dem Gasstrom gemäß (1) enthaltene Ether ist.

Somit werden gemäß der vorliegenden Erfindung Ausführungsformen des Verfahrens zur Umwandlung von Ethern zu Olefinen bevorzugt, bei welchen der Gasstrom gemäß (1) ein oder mehrere Di-(C₁-C₃)alkylether, bevorzugt ein oder mehrere Etherverbindungen ausgewählt aus der Gruppe bestehend aus Dimethylether, Diethylether, Ethylmethylether, Di-n-propylether, Diisopropylether, und Mischungen von zwei oder mehr davon, weiter bevorzugt aus der Gruppe bestehend aus Dimethylether, Diethylether, Ethylmethylether und Mischungen von zwei oder mehr davon, wobei der Gasstrom gemäß (1) weiter bevorzugt Dimethylether enthält.

Was andererseits den Gehalt an Ethern in dem Gasstrom gemäß (1) im erfindungsgemäßen Verfahren zur Umwandlung von Ethern zu Olefinen betrifft, so besteht gemäß vorliegender Erfindung auch hier keinerlei Einschränkung, sofern beim Inkontaktbringen des Gasstroms in (2) mit einem Katalysator gemäß vorliegender Erfindung mindestens ein Ether zu mindestens einem Olefin umgesetzt werden kann. Gemäß bevorzugter Ausführungsformen liegt der Gehalt an Ethern in dem Gasstrom gemäß (1) im Bereich von 30 bis 100 Vol.-% bezogen auf das Gesamtvolumen, wobei sich der Gehalt insbesondere auf einen Gasstrom bei einer Temperatur im Bereich von 200 bis 700°C und bei einem Druck von 101,3 kPa bezieht, bevorzugt bei einer Temperatur im Bereich von 250 bis 650°C, weiter bevorzugt von 300 bis 600°C, weiter bevorzugt von 350 bis 560°C, weiter bevorzugt von 400 bis 540°C, weiter bevorzugt von 430 bis 520°C, und weiter bevorzugt im Bereich von 450 bis 500°C und bei einem Druck von 101,3 kPa. Gemäß vorliegender Erfindung ist es weiter bevorzugt, dass der Gehalt an Ethern in dem Gasstrom gemäß (1) im Bereich von 30 bis 99 Vol.-% liegt, weiter bevorzugt von 30 bis 95 Vol.-%, weiter bevorzugt von 30 bis 90 Vol.-%, weiter bevorzugt von 30 bis 80 Vol.-%, weiter bevorzugt von 30 bis 70 Vol.-%, weiter bevorzugt von 30 bis 60 Vol.-% und weiter bevorzugt von 30 bis 50 Vol.-%. Gemäß besonders bevorzugten Ausführungsformen des erfindungsgemäßen Verfahrens zur Umwandlung von Ethern zu Olefinen liegt der Gehalt an Ethern in dem Gasstrom gemäß (1) im Bereich von 30 bis 45 Vol.-%.

Somit werden gemäß der vorliegenden Erfindung Ausführungsformen des Verfahrens zur Umwandlung von Ethern zu Olefinen bevorzugt, bei welchen der Gehalt an Ethern in dem Gasstrom gemäß (1) im Bereich von 30 bis 100 Vol.-% bezogen auf das Gesamtvolumen liegt, bevorzugt von 30 bis 99 Vol.-%, weiter bevorzugt von 30 bis 95 Vol.-%, weiter bevorzugt von 30 bis 90 Vol.-%, weiter bevorzugt von 30 bis 80 Vol.-%, weiter bevorzugt von 30 bis 70 Vol.-%, weiter bevorzugt von 30 bis 60 Vol.-%, weiter bevorzugt von 30 bis 50 Vol.-%, und weiter bevorzugt von 30 bis 45 Vol.-%.

Gemäß vorliegender Erfindung besteht prinzipiell keinerlei Einschränkung hinsichtlich der Zusammensetzung des Gasstroms in (1) vorausgesetzt mindestens ein der im Gasstrom enthaltenen Ether kann zu mindestestens einem Olefin umgesetzt werden im erfindungsgemäßen Verfahren. Somit besteht auch keinerlei Einschränkung hinsichtlich der Herkunft des in (1) bereitgestellten Gasstroms, sofern die zuvorgenannten Bedingung der Umwandlung mindestens eines Ethers zu mindestens einem Olefin entsprechend erfüllt ist. Dementsprechend kann der Gasstrom prinzipiell aus einem oder mehreren Ethern und einer oder mehreren zusätzlichen Verbindungen zu einem Gasstrom zusammengesetzt werden. Gemäß besonders bevorzugten Ausführungsformen des erfindungsgemäßen Verfahrens zur chemischen Umwandlung von Ethern zu Olefinen stammt der in (1) bereitgestellte Gasstrom aus mindestens einer Vorreaktion, bevorzugt aus der chemischen Umwandlung von einem oder mehreren Alkoholen zu einem oder mehreren Ethern, wobei der eine oder die mehreren Alkohole bevorzugt aus der Gruppe der aliphatischen Alkohole ausgewählt sind. Gemäß weiter bevorzugten Ausführungsformen stammt zumindest ein Teil der in (1) bereitgestellte Gasstrom aus der chemischen Umwandlung von einem oder mehreren aliphatischen (C₁-C₆) Alkoholen und Mischungen von zwei oder mehr davon, weiter bevorzugt aus der Umwandlung von einem oder mehreren aliphatischen (C₁-C₄) Alkoholen und Mischungen von zwei oder mehr davon, weiter bevorzugt aus der chemischen Umwandlung einer oder mehrerer aliphatischer Alkohole ausgewählt aus der Gruppe bestehend aus Methanol, Ethanol, n-Propanol, Isopropanol, Butanol und Mischungen von zwei oder mehr davon, weiter bevorzugt aus der Gruppe bestehend aus Methanol, Ethanol, n-Propanol und Mischungen von zwei oder mehr davon, wobei besonders bevorzugt der in (1) bereitgestellte Gasstrom einer Vorreaktion von Methanol und/oder Ethanol entstammt und weiter bevorzugt Methanol zumindest teilweise zu einem oder mehreren Di-(C₁-C₂)alkylethern umgesetzt wird, bevorzugt zu einem oder mehreren Di-(C₁-C₂)alkylethern ausgewählt aus der Gruppe bestehend aus Dimethylether, Diethylether, Ethylmethylether und Mischungen von zwei oder mehr davon. So entstammt der in (1) bereitgestellte Gasstrom gemäß einer besonders bevorzugten Ausführungsform einer Vorreaktion der Umwandlung von Methanol zu Dimethylether.

Bei den besonders bevorzugten Ausführungsformen des erfindungsgemäßen Verfahrens, bei welchen der in (1) bereitgestellte Gasstrom einer Vorreaktion von einem oder mehreren Alkoholen entstammt, besteht prinzipiell keinerlei besondere Einschränkung hinsichtlich der Reaktion und damit des Reaktionsprodukts der Umwandlung von einem oder mehreren Alkoholen, vorausgesetzt diese führt zu einem Gasstrom enthaltend ein oder mehrere Ether, welche bei Inkontaktbringen in (2) mit einem Katalysator gemäß der vorliegenden Erfindung die Umwandlung mindestens eines der Ether zu mindestens einem Olefin ermöglichen. Gemäß diesen besonderen Ausführungsformen ist es weiter bevorzugt, dass die Vorreaktion zur Umwandlung mindestens eines Alkohols zu mindestens einem Ether und insbesondere zu mindestens einem Dialkylether führt, wobei es sich bei der Vorreaktion besonders bevorzugt um eine Dehydratisierung handelt, bei welcher Wasser als Kuppelprodukt zu einem oder mehreren Dialkylethern anfällt. Gemäß den besonderen und bevorzugten Ausführungsformen der vorliegenden Erfindung, bei welchen der in (1) bereitgestellte Gasstrom einer Vorreaktion entstammt, ist es gemäß dem erfindungsgemäßen Verfahren besonders bevorzugt, dass ein solcher einer Vorreaktion entstammender Gasstrom direkt und ohne Aufarbeitung dem erfindungsgemäßen Verfahren in Schritt (1) zugeführt wird.

Somit werden gemäß der vorliegenden Erfindung Ausführungsformen des Verfahrens zur Umwandlung von Ethern zu Olefinen bevorzugt, bei welchen der Gasstrom gemäß (1) aus einer Vorstufe erhältlich ist, bevorzugt aus einer Vorstufe der Dehydratisierung von einem oder mehreren aliphatischen Alkoholen, bevorzugt eines oder mehrerer (C₁-C₆) Alkoholen, weiter bevorzugt eines oder mehrerer (C₁-C₄) Alkoholen, weiter bevorzugt eines oder mehrerer aliphatischer Alkohole ausgewählt aus der Gruppe bestehend aus Methanol, Ethanol, n-Propanol, Isopropanol, Butanol und Mischungen von zwei oder mehr davon, weiter bevorzugt aus einer Vorstufe der Dehydratisierung von Methanol und/oder Ethanol, bevorzugt von Methanol.

Bezüglich der Dehydratisierung welche bevorzugt in der Vorstufe zur Bereitstellung des Gasstroms in (1) gemäß besonders bevorzugten Ausführungsformen des erfindungsgemäßen Verfahrens durchgeführt wird besteht wiederum keinerlei Einschränkungen hinsichtlich der Art und Weise wie diese durchgeführt wird, vorausgesetzt mindestens ein Alkohol und bevorzugt mindestens ein aliphatischer Alkohol zu mindestens einem Ether chemisch umgewandelt wird. Gemäß bevorzugten Ausführungsformen handelt es sich bei der Dehydratisierung zumindest teilweise um eine katalytische Dehydratisierung, wobei prinzipiell keinerlei Einschränkung gilt hinsichtlich des hierfür verwendeten Katalysators, vorausgesetzt dieser ist in der Lage unter den gewählten Bedingungen der Vorreaktion mindestens einen Alkohol und bevorzugt mindestens einen aliphatischen Alkohol zu mindestens einem Ether katalytisch umzuwandeln, bevorzugt unter der gleichzeitigen Bildung von Wasser. Gemäß besonders bevorzugten Ausführungsformen des erfindungsgemäßen Verfahrens wird ein heterogener Katalysator für die bevorzugte Dehydratisierung als Vorreaktion verwendet, wobei der Katalysator bevorzugt in fester Form vorliegt und bevorzugt saure Zentren aufweist, wobei diese bevorzugt zum Teil als Lewis-saure Zentren vorliegen. Somit wird gemäß diesen besonders bevorzugten Ausführungsformen des erfindungsgemäßen Verfahrens beispielsweise Aluminiumoxid als heterogener Katalysator für die Vorreaktion verwendet, wobei gemäß einer besonders bevorzugten Ausführungsform gamma-Aluminiumoxid als heterogener Katalysator für die Dehydratisierung dient.

Hinsichtlich der Reaktionsbedingungen welche für die Dehydratisierung gemäß den bevorzugten Ausführungsformen des erfindungsgemäßen Verfahrens gewählt werden, so bestehen auch in dieser Hinsicht prinzipiell keine Beschränkungen, vorausgesetzt mindestens ein Alkohol und bevorzugt mindestens ein aliphatischer Alkohol zu mindestens einem Ether chemisch umgewandelt werden kann. Bezüglich der für die Dehydratisierung gewählte Temperatur kann somit jede hierfür geeignete Temperatur eingestellt werden, wobei bei der Dehydratisierung gemäß den besonderen oder bevorzugten Ausführungsformen in welchen ein heterogener Katalysator eingesetzt wird die Temperatur für die Vorreaktion bevorzugt im Bereich von 100 bis 600°C liegt, weiter bevorzugt von 150 bis 500°C, weiter bevorzugt von 200 bis 400°C, weiter bevorzugt von 230 bis 350°C, weiter bevorzugt von 250 bis 300°C, und weiter bevorzugt von 270 bis 280°C.

Was die anderen Komponenten in dem Gasstrom gemäß (1) im erfindungsgemäßen Verfahrens betrifft, besteht prinzipiell keinerlei Einschränkung, vorausgesetzt der Gasstrom ist insgesamt zur Umwandlung mindestens eines der Ether zu mindestens einem Olefin in Schritt (2) beim Inkontaktbringen mit einem Katalysator gemäß vorliegender Erfindung geeignet. Weiterhin können beispielsweise neben dem einem oder den mehreren Ethern im Gasstrom gemäß (1) ein oder mehrere Inertgase darin enthalten sein, wie beispielsweise ein oder mehrere Edelgase, Stickstoff, Wasser und Mischungen von zwei oder mehr davon. Gemäß besonderen Ausführungsformen der vorliegenden Erfindung enthält der Gasstrom gemäß (1) des erfindungsgemäßen Verfahrens neben dem einen oder den mehreren Ethern Wasser, wobei dies insbesondere für die besonderen und bevorzugten Ausführungsformen der vorliegenden Erfindung gilt, bei denen der Gasstrom gemäß (1) aus einer Vorstufe der Dehydratisierung erhalten wird.

Hinsichtlich jener bevorzugten Ausführungsformen, in welchen neben dem einen oder den mehreren Ethern Wasser im Gasstrom gemäß (1) enthalten ist, besteht prinzipiell keine Beschränkung hinsichtlich des Gehalts an Wasser, welcher darin enthalten sein kann, sofern die Umwandlung von mindestens einem Ether im Gasstrom zu mindestens einem Olefin in Schritt (2) des Inkontaktbringens des Gasstroms mit einem Katalysator gemäß der vorliegenden Erfindung erfolgen kann. Gemäß dieser bevorzugten Ausführungsformen ist es jedoch bevorzugt, dass der Gehalt an Wasser in dem Gasstrom im Bereich von 5 bis 60 Vol.-% bezogen auf das Gesamtvolumen liegt, wobei der Gehalt an Wasser besonders bevorzugt im Bereich von 10 bis 55 Vol.-% liegt, weiter bevorzugt von 20 bis 50 Vol.-% und weiter bevorzugt von 30 bis 45 Vol.-%.

Somit werden gemäß der vorliegenden Erfindung Ausführungsformen des Verfahrens zur Umwandlung von Ethern zu Olefinen bevorzugt, bei welchen Wasser in dem Gasstrom gemäß (1) enthalten ist, bevorzugt im Bereich von 5 bis 60 Vol.-% bezogen auf das Gesamtvolumen liegt, bevorzugt von 10 bis 55 Vol.-%, weiter bevorzugt von 20 bis 50 Vol.-%, und weiter bevorzugt von 30 bis 45 Vol.-%.

Hinsichtlich der Art und Weise des Inkontaktbringens des Gasstroms mit einem Katalysator gemäß vorliegender Erfindung in Schritt (2) des erfindungsgemäßen Verfahrens zur Umwandlung von Ethern zu Olefinen besteht prinzipiell keinerlei Einschränkung, sofern die Umwandlung von mindestens einem Ether zu mindestens einem Olefin realisiert werden kann. Dies gilt beispielsweise für die Temperatur, bei welcher das Inkontaktbringen (2) stattfindet. Somit findet das Inkontaktbringen in Schritt (2) des erfindungsgemäßen Verfahrens bei einer Temperatur im Bereich von 430 bis 520 °C statt. Gemäß bevorzugten Ausführungsformen der vorliegenden Erfindung wird das Inkontaktbringen gemäß (2) des erfindungsgemäßen Verfahrens bei einer Temperatur im Bereich von 450 bis 500 °C durchgeführt.

Somit werden gemäß der vorliegenden Erfindung Ausführungsformen des Verfahrens zur Umwandlung von Ethern zu Olefinen bevorzugt, bei welchen das Inkontaktbringen gemäß (2) bei einer Temperatur im Bereich von 430 bis 520°C, und bevorzugt von 450 bis 500°C erfolgt.

Gleiches gilt entsprechend für den Druck, bei welchem der Gasstrom in Schritt (2) des erfindungsgemäßen Verfahrens mit dem Katalysator gemäß vorliegender Erfindung in Kontakt gebracht wird. Somit kann das Inkontaktbringen prinzipiell bei jedem beliebigen Druck stattfinden, vorausgesetzt, dieser erlaubt die Umwandlung von mindestens einem Ether zu mindestens einem Olefin durch das Inkontaktbringen des Gasstroms mit dem Katalysator. Somit kann der Druck beispielsweise beim Inkontaktbringen in Schritt (2) im Bereich von 0,1 bis 10 bar liegen, wobei der Druck gemäß vorliegender Anmeldung den absoluten Druck kennzeichnet, so dass ein Druck von 1 bar beim Inkontaktbringen entsprechend dem Normaldruck von 1,03 kPa entspricht. Gemäß vorliegender Erfindung findet das Inkontaktbringen in Schritt (2) bevorzugt bei einem Druck von 0,3 bis 7 bar, weiter bevorzugt von 0,5 bis 5 bar, weiter bevorzugt von 0,7 bis 3 bar, weiter bevorzugt von 0,8 bis 2,5 bar und weiter bevorzugt von 0,9 bis 2,2 bar statt. Gemäß besonders bevorzugten Ausführungsformen des erfindungsgemäßen Verfahrens zur Umwandlung von Ethern zu Olefinen findet das Inkontaktbringen in Schritt (2) bei einem Druck von 1 bis 2 bar statt.

Somit werden gemäß der vorliegenden Erfindung Ausführungsformen des Verfahrens zur Umwandlung von Ethern zu Olefinen bevorzugt, bei welchen das Inkontaktbringen gemäß (2) bei einem Druck im Bereich von 0,1 bis 10 bar, bevorzugt von 0,3 bis 7 bar, weiter bevorzugt von 0,5 bis 5 bar, weiter bevorzugt von 0,7 bis 3 bar, weiter bevorzugt von 0,8 bis 2,5 bar, weiter bevorzugt von 0,9 bis 2,2 bar, und weiter bevorzugt von 1 bis 2 bar erfolgt.

Weiterhin bestehen keine besonderen Einschränkungen hinsichtlich der Art der Durchführung des erfindungsgemäßen Verfahrens zur Umwandlung von Ethern zu Olefinen, so dass sowohl ein kontinuierliches als auch ein nichtkontinuierliches Verfahren verwendet werden kann, wobei das nichtkontinuierliche Verfahren beispielsweise in Form eines Batch-Verfahrens durchgeführt werden kann. Gemäß vorliegender Erfindung ist es jedoch bevorzugt, das erfindungsgemäße Verfahren für die Umwandlung von Ethern als kontinuierliches Verfahren zu führen. Somit werden gemäß der vorliegenden Erfindung Ausführungsformen des Verfahrens zur Umwandlung von Ethern zu Olefinen bevorzugt, bei welchen das Verfahren ein kontinuierliches Verfahren ist.

Hinsichtlich dieser bevorzugten Ausführungsformen eines kontinuierlichen Verfahrens bestehen keinerlei Einschränkungen hinsichtlich der gewählten Raumgeschwindigkeit, vorausgesetzt die Umwandlung von einem Ether zu einem Olefin kann erfolgen. Somit können beispielsweise Raumgeschwindigkeiten beim Inkontaktbringen in Schritt (2) gewählt werden, welche im Bereich von 0,5 bis 50 h⁻¹ liegen, wobei bevorzugt Raumgeschwindigkeiten (WHSV = weight hourly space velocity wird als Verhältnis von Oxygenat-Eduktstrom in kg/h zur Menge von Zeolith im Reaktor in kg errechnet) von 1 bis 30 h⁻¹ gewählt werden, weiter bevorzugt von 3 bis 25 h⁻¹, weiter bevorzugt von 5 bis 20 h⁻¹, weiter bevorzugt von 7 bis 15 h⁻¹ und weiter bevorzugt von 8 bis 12 h⁻¹. Gemäß besonders bevorzugten Ausführungsformen des erfindungsgemäßen Verfahrens zur Umwandlung von Ethern werden Raumgeschwindigkeiten für das Inkontaktbringen des Gasstroms in Schritt (2) im Bereich von 9 bis 11 h⁻¹ gewählt.

Somit werden gemäß der vorliegenden Erfindung Ausführungsformen des Verfahrens zur Umwandlung von Ethern zu Olefinen bevorzugt, bei welchen die Raumgeschwindigkeit beim Inkontaktbringen gemäß (2) im Bereich von 0,5 bis 50 h⁻¹, bevorzugt von 1 bis 30 h⁻¹, weiter bevorzugt von 3 bis 25 h⁻¹, weiter bevorzugt von 5 bis 20 h⁻¹, weiter bevorzugt von 7 bis 15 h⁻¹, weiter bevorzugt von 8 bis 12 h⁻¹, und weiter bevorzugt von 9 bis 11 h⁻¹ liegt.

Wie weiter oben beschrieben und in den Beispielen der vorliegenden Anmeldung gezeigt, können bei einem wie in der vorliegenden Anmeldung beschriebenen Verfahren zur Umwandlung von Ethern besonders hohe Standzeiten mit dem erfindungsgemäßen Katalysator erzielt werden, insbesondere hinsichtlich der besonderen und bevorzugten Ausführungsformen des erfindungsgemäßen Verfahrens. So wurde überraschenderweise festgestellt, dass durch die Verwendung eines Katalysators gemäß der vorliegenden Erfindung die Standzeit des Katalysators, bis zu welcher das Verfahren zur Regenerierung des Katalysators unterbrochen werden muss, zumindest hinsichtlich der Verwendung dieser Katalysatorcharge gegenüber der Verwendung von Katalysatoren gemäß dem Stand der Technik erheblich erhöht werden kann. So ist es gemäß vorliegender Erfindung besonders bevorzugt, für die Durchführung des Verfahrens zur Umwandlung von Ethern zu Olefinen bei einer der besonderen oder bevorzugten Raumgeschwindigkeiten, wie in der vorliegenden Anmeldung beschrieben, hohe Standzeiten zu wählen.

Somit werden Standzeiten bevorzugt, welche im Bereich von 15 bis 200 h liegen, weiter bevorzugt im Bereich von 20 bis 150 h, weiter bevorzugt von 25 bis 100 h, weiter bevorzugt von 30 bis 80 h, weiter bevorzugt von 35 bis 70 h, weiter bevorzugt von 40 bis 65 h, weiter bevorzugt von 45 bis 60 h und weiter bevorzugt von 50 bis 55 h. Insbesondere, bezogen auf die besonderen und bevorzugten Raumgeschwindigkeiten, bei welchen das erfindungsgemäßen Verfahren durchgeführt wird, werden somit beispielsweise Standzeiten von 15 bis 200 h bei einer Raumgeschwindigkeit im Bereich von 0,5 bis 50 h⁻¹ bevorzugt. Weiter bevorzugt ist eine Standzeit von 20 bis 150 h bei einer Raumgeschwindigkeit von 1 bis 30 h⁻¹, weiter bevorzugt eine Standzeit von 25 bis 100 h bei einer Raumgeschwindigkeit von 1 bis 30 h⁻¹, weiter bevorzugt eine Standzeit von 30 bis 80 h bei einer Raumgeschwindigkeit von 3 bis 25 h⁻¹, weiter bevorzugt eine Standzeit von 35 bis 70 h bei einer Raumgeschwindigkeit im Bereich von 5 bis 20 h⁻¹, weiter bevorzugt eine Standzeit von 40 bis 65 h bei einer Raumgeschwindigkeit im Bereich von 7 bis 15 h⁻¹ und weiter bevorzugt von 45 bis 60 h bei einer Raumgeschwindigkeit von 8 bis 12 h⁻¹. Gemäß einer besonders bevorzugten Ausführungsform des erfindungsgemäßen Verfahrens wird eine Standzeit des Katalysators gewählt, während der das kontinuierliche Verfahren ohne Unterbrechung durchgeführt wird, welche im Bereich von 50 bis 55 h bei einer Raumgeschwindigkeit von 9 bis 11 h⁻¹ liegt. Gemäß der vorliegenden Erfindung beziehen sich die besonderen und bevorzugten Ausführungsformen hinsichtlich der gewählten Standzeit und insbesondere der gewählten Standzeiten in Kombination mit bestimmten Raumgeschwindigkeiten bevorzugt auf einen Mindestumsatz des einen oder der mehreren Ether, welche im Gasstrom gemäß (1) des erfindungsgemäßen Verfahrens enthalten sind, bei dessen dauerhaften Unterschreitung die Regenerierung des Katalysators anschließend durchgeführt wird. Gemäß vorliegender Erfindung besteht keine besondere Einschränkung hinsichtlich des gewählten Mindestumsatzes, wobei dieser bevorzugt einen Vollumsatz des einen oder der mehreren Ether, welche im Gasstrom gemäß (1) des erfindungsgemäßen Verfahrens enthalten sind, während der Standzeit des Katalysators erlaubt. So wird gemäß bevorzugten Ausführungsformen der vorliegenden Erfindung ein Mindestumsatz von 60% des einen oder der mehreren Ether, welche im Gasstrom gemäß (1) des erfindungsgemäßen Verfahrens enthalten sind, gewählt, bei dessen dauerhaften Unterschreitung die Regenerierung des Katalysators durchgeführt wird, bevorzugt ein Mindestumsatz von 70% oder mehr, weiter bevorzugt von 80% oder mehr, weiter bevorzugt von 85% oder mehr, weiter bevorzugt von 90% oder mehr, weiter bevorzugt von 95% oder mehr, weiter bevorzugt von 97% oder mehr, weiter bevorzugt von 98% oder mehr, und weiter bevorzugt von 99% oder mehr des einen oder der mehreren Ether, welche im Gasstrom gemäß (1) des erfindungsgemäßen Verfahrens enthalten sind.

Somit werden gemäß der vorliegenden Erfindung Ausführungsformen des Verfahrens zur Umwandlung von Ethern zu Olefinen weiter bevorzugt, bei welchen die Standzeit des beschichteten Trägersubstrats als Katalysator, während der das kontinuierliche Verfahren ohne Unterbrechung durchgeführt wird, im Bereich von 15 bis 200 h, bevorzugt von 20 bis 150 h, weiter bevorzugt von 25 bis 100 h, weiter bevorzugt von 30 bis 80 h, weiter bevorzugt von 35 bis 70 h, weiter bevorzugt von 40 bis 65 h, weiter bevorzugt von 45 bis 60 h, und noch weiter bevorzugt von 50 bis 55 h liegt.

Gemäß der vorliegenden Erfindung kann der Katalysator prinzipiell regeneriert werden, um von neuem in das erfindungsgemäße Verfahren eingesetzt zu werden. Bezüglich der Regenerierung des Katalysators bestehen keinerlei Einschränkungen, sofern die Regenerierung zu einer zumindest partiellen Wiederherstellung seiner ursprünglichen Aktivität in der Umwandlung von Oxygenaten zu Olefinen führt. Nach einer besonders bevorzugten Ausführungsform des erfindungsgemäßen Verfahrens wird der Katalysator durch thermische Behandlung und insbesondere durch Kalzinierung regeneriert und wieder in das Verfahren eingesetzt.

Somit werden gemäß der vorliegenden Erfindung Ausführungsformen des Verfahrens zur Umwandlung von Ethern zu Olefinen bevorzugt, bei welchen das Verfahren die weiteren Schritte umfasst
(3) Kalzinierung des Katalysators zur Regenerierung, wobei die Kalzinierung bei einer Temperatur im Bereich von 450 bis 550 °C erfolgt;
(4) Bereitstellen eines Gasstroms enthaltend ein oder mehrere Ether;
(5) Inkontaktbringen des in (4) bereitgestellten Gasstroms mit dem regenerierten Katalysator.

Hinsichtlich der Dauer der Kalzinierung des Katalysators in (3) besteht prinzipiell keinerlei Einschränkung, sofern diese zu einer zumindest partiellen Wiederherstellung der ursprünglichen katalytischen Aktivität bei der Umwandlung von Oxygenaten zu Olefinen beiträgt. Die Kalzinierung wird bei einer Temperatur im Bereich von 450 bis 550°C, bevorzugt im Bereich von 475 bis 525°C, durchgeführt. Bezüglich der Dauer der Kalzinierung kann diese beispielsweise für eine Dauer von 0,25 bis 30 h durchgeführt werden, wobei die Dauer Kalzinierung bevorzugt im Bereich von 0,5 bis 20 h liegt, weiter bevorzugt von 1 bis 15 h, weiter bevorzugt von 1,5 bis 12 h, weiter bevorzugt von 2 bis 10 h, weiter bevorzugt von 3 bis 8 h, weiter bevorzugt von 3,5 bis 7 h, weiter bevorzugt von 4 bis 6 h, und weiter bevorzugt von 4,5 bis 5,5 h.

Die Kalzinierung in (3) zur Regenerierung des Katalysators kann prinzipiell in jeder geeigneten Atmosphäre durchgeführt werden, vorausgesetzt eine zumindest teilweisen Wiederherstellung der ursprünglichen Aktivität kann erreicht werden. Somit kann die Kalzinierung beispielsweise in Sauerstoff oder in einer Sauerstoff enthaltenden Atmosphäre wie Luft oder in einem Gemisch von Sauerstoff und einem inerten Gas wie Stickstoff und/oder ein oder mehrere Edelgase durchgeführt werden. Gemäß bevorzugten Ausführungsformen wird die Kalzinierung in (3) in Luft oder in einem Gemisch von Sauerstoff und einem inerten Gas durchgeführt, wobei die Kalzinierung in (3) besonders bevorzugt in Luftatmosphäre erfolgt.

Grundsätzlich kann der Katalysator nach den bevorzugten Ausführungsformen nach welchen dieser regeneriert wird (3) zu jedem geeigneten Zeitpunkt des Verfahrens regeneriert werden, vorausgesetzt diese führt zu einer zumindest teilweisen Wiederherstellung der anfänglichen Aktivität, als der eingesetzte Katalysator noch nicht in der Umsetzung von Oxygenaten zu Olefinen eingesetzt worden war und insbesondere als dieser noch frisch oder frisch regeneriert war. Somit kann die Kalzinierung in (3) zur Regenerierung des Katalysator beispielsweise dann durchgeführt werden, wenn der Methanol-Umsatz in dem Verfahren zur Umwandlung von Oxygenaten zu Olefinen unter 70% fällt, wobei die Regenerierung bevorzugt dann durchgeführt wird, wenn der Methanol-Umsatz in der Reaktion auf 70% fällt, und weiter bevorzugt auf 75%, weiter bevorzugt auf 80%, weiter bevorzugt auf 85%, weiter bevorzugt auf 90%, weiter bevorzugt auf 95%, und weiter bevorzugt auf 97% fällt.

Bezüglich des auf die Kalzinierung in (3) folgenden Schrittes der Bereitstellung eines Gasstroms enthaltend ein oder mehrere Ether in (4) sowie des Inkontaktbringens des Gasstroms mit dem regenerierten Katalysator in (5), so werden diese Schritte prinzipiell analog den Schritten (1) und (2) des erfindungsgemäßen Verfahrens durchgeführt, und insbesondere gemäß den besonderen und bevorzugten Ausführungsformen von den Schritten (1) und (2) wie in der vorliegenden Anmeldung definiert. Somit gelten alle besonderen und bevorzugten Ausführungsformen für Schritt (1) in gleicher Weise für Schritt (4) und, unabhängig davon, auch alle besonderen und bevorzugten Ausführungsformen für Schritt (2) in gleicher Weise für Schritt (5).

Was die bevorzugten Ausführungsformen des erfindungsgemäßen Verfahrens betrifft bei welchen der Katalysator einer Kalzinierung zur Regenerierung des Katalysators unterworfen wird, so wurde ganz überraschender Weise festgestellt das die Regenerierung zu einer weiteren Verbesserung der Standzeit des Katalysators führte, obwohl man den gegenläufigen Effekt bei einem bereits verwendeten Katalysator erwarten würde. Umso überraschender war dann noch der zusätzliche Effekt, dass die Selektivitäten des Katalysators hin zu C₃- und C₄-Olefinen durch die Kalzinierung deutlich gesteigert werden konnten. Somit dient die bevorzugte Kalzinierung des Katalysators in (3) nicht nur dessen Regenerierung sondern führt unerwarteter Weise zur Steigerung sowohl der Standzeit als auch der Selektivität des Katalysators hin zu C₃- und C₄-Olefinen als Produkte der Umwandlung von Oxygenaten, insbesondere gemäß den besonderen und bevorzugten Ausführungsformen des erfindungsgemäßen Verfahrens.

Schließlich können die Schritte (3) bis (5) der bevorzugten Ausführungsformen des erfindungsgemäßen Verfahrens beliebig wiederholt werden, so dass diese beispielsweise ein bis 1000 Mal wiederholt werden können. Gemäß der vorliegenden Erfindung ist es jedoch bevorzugt, dass die Schritte (3) bis (5) 5 bis 800 Mal wiederholt werden, weiter bevorzugt 10 bis 700 Mal, weiter bevorzugt 15 bis 600 Mal, weiter bevorzugt 20 bis 500 Mal, weiter bevorzugt 25 bis 400 Mal, und weiter bevorzugt 30 bis 300 Mal wiederholt werden.

Der im Verfahren gemäß der vorliegenden Erfindung verwendete Katalysator kann prinzipiell auf jede geeignete Art und Weise hergestellt werden, vorausgesetzt dieser enthält einen oder mehrere Zeolithe vom MFI-Strukturtyp, welche in einer auf ein Trägersubstrat aufgebrachten Schicht enthalten sind gemäß der vorliegenden Erfindung und insbesondere gemäß einer der besonderen und bevorzugten Ausführungsformen der Erfindung, wie in der vorliegenden Anmeldung beschrieben. Gemäß der vorliegenden Erfindung ist der Katalysator zur Anwendung im erfindungsgemäßen Verfahren bevorzugt gemäß einem der in der vorliegenden Anmeldung beschriebenen Verfahren zu dessen Herstellung erhältlich, bevorzugt gemäß einem der besonderen oder bevorzugten Verfahren zu dessen Herstellung, wobei dieser gemäß besonders bevorzugten Ausführungsformen der vorliegenden Erfindung gemäß einem der in der vorliegenden Anmeldung beschriebenen Verfahren erhalten wird, bevorzugt gemäß einem der besonderen oder bevorzugten Verfahren zu dessen Herstellung.

Somit werden gemäß der vorliegenden Erfindung Ausführungsformen des Verfahrens zur Umwandlung von Ethern zu Olefinen weiter bevorzugt, bei welchen der Katalysator, und insbesondere der der Katalysator gemäß einer der besonderen oder bevorzugten Ausführungsformen des erfindungsgemäßen Verfahrens, erhältlich ist nach einem Verfahren umfassend
(i) Bereitstellen des Trägersubstrats und des einen oder der mehreren Zeolithe vom MFI-Strukturtyp;
(ii) Herstellen eines Gemischs, enthaltend den einen oder die mehreren Zeolithe vom MFI-Strukturtyp und ein oder mehrere Lösungsmittel;
(iii) Homogeniseren des in (ii) erhaltenen Gemischs;
(iv) Beschichten des Trägersubstrats mit dem in (iii) erhaltenen homogenisierten Gemisch;
(v) optionales Trocknen des in (iv) erhaltenen beschichteten Trägersubstrats;
(vi) optionales Kalzinieren des in (iv) oder (v) erhaltenen beschichteten Trägersubstrats.

Bezüglich des Verfahrens zur Herstellung des im erfindungsgemäßen Verfahren verwendeten Katalysators, insbesondere gemäß der in der vorliegenden Anmeldung beschriebenen besonderen und bevorzugten Ausführungsformen, besteht prinzipiell keinerlei Einschränkung hinsichtlich der Beschaffenheit und insbesondere der Partikelgrößen und/oder Morphologien des einen oder der mehreren in Schritt (i) bereitgestellten Zeolithe vom MFI-Strukturtyp. Je nach Partikelgröße der bereitgestellten Zeolithe in Schritt (i) werden jedoch optional ein oder mehrere Schritte während des erfindungsgemäßen Verfahrens durchgeführt, bevorzugt vor dem Bereitstellen des einen oder der mehreren Zeolithe in (i) oder nach dem Herstellen des Gemischs in Schritt (ii), um den einen oder die mehreren Zeolithe auf eine bevorzugte Partikelgröße zu bringen. In diesem Zusammenhang besteht zunächst keine besondere Einschränkung hinsichtlich der Partikelgröße des einen oder der mehreren Zeolithe, vorausgesetzt, diese ist für die Durchführung der weiteren Schritte im erfindungsgemäßen Verfahren, insbesondere gemäß den besonderen und bevorzugten Ausführungsforme der vorliegenden Erfindung, geeignet, wobei die Partikelgröße insbesondere zur Durchführung des Beschichtens in Schritt (iv) geeignet sein soll, insbesondere in Abhängigkeit von der Art und Form des verwendeten Trägersubstrats gemäß der vorliegenden Erfindung und insbesondere gemäß den besonderen oder bevorzugten Ausführungsformen des Trägersubstrats wie in der vorliegenden Anmeldung beschrieben. Somit werden bei bestimmten Ausführungsformen des erfindungsgemäßen Verfahrens ein oder mehrere Schritte vor dem Bereitstellen des einen oder der mehreren Zeolithe in (i) oder nach dem Herstellen des Gemischs in Schritt (ii), bevorzugt nach dem Herstellen des Gemischs in Schritt (ii) und besonders bevorzugt in Schritt (iii) des Homogenisierens des in (ii) erhaltenen Gemischs, durchgeführt um den einen oder die mehreren Zeolithe vom MFI-Strukturtyp auf eine Partikelgröße D₅₀ im Bereich von 0,01 bis 200 µm zu bringen. Nach weiter bevorzugten Ausführungsformen des erfindungsgemäßen Verfahrens werden der eine oder die mehreren Zeolithe nach einem oder mehreren der zuvor genannten Schritte in einem oder mehreren Schritten auf eine Partikelgröße D₅₀ im Bereich von 0,03 bis 150 µm, weiter bevorzugt von 0,05 bis 100 µm, weiter bevorzugt von 0,1 bis 50 µm, weiter bevorzugt von 0,3 bis 30 µm und noch weiter bevorzugt von 0,4 bis 20 µm gebracht. Nach noch weiteren bevorzugten Ausführungsformen des erfindungsgemäßen Verfahrens werden der eine oder die mehreren Zeolithe nach dem Herstellen des Gemischs in Schritt (ii) und bevorzugt in Schritt (iii) des Homogenisierens des in (ii) erhaltenen Gemischs in einem oder mehreren Schritten auf eine Partikelgröße D₅₀ im Bereich von 0,5 bis 15 µm gebracht. Hinsichtlich der Anzahl der Schritte und der Art und Weise, auf welche der eine oder die mehreren Zeolithe auf eine besondere oder bevorzugte Partikelgröße D₅₀ gebracht werden, bestehen gemäß vorliegender Erfindung keinerlei Einschränkungen, so dass jedes geeignete Verfahren hierfür prinzipiell verwendet werden kann. Gemäß der vorliegenden Erfindung werden der eine oder die mehreren Zeolithe jedoch bevorzugt einem oder mehreren Schritten des Mahlens vor dem Bereitstellen des einen oder der mehreren Zeolithe in (i) oder nach dem Herstellen des Gemischs in Schritt (ii) unterzogen, bevorzugt nach dem Herstellen des Gemischs in Schritt (ii), wobei der eine oder die mehreren Zeolithe besonders bevorzugt durch den Vorgang des Homogenisierens in Schritt (iii), insbesondere nach den besonderen und bevorzugten Ausführungsformen der vorliegenden Erfindung, auf eine der besonderen oder bevorzugten Partikelgrößen D₅₀ gebracht wird.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen vor dem Bereitstellen des einen oder der mehreren Zeolithe in (i) oder nach dem Herstellen des Gemischs in Schritt (ii), bevorzugt nach dem Herstellen des Gemischs in Schritt (ii) und besonders bevorzugt in Schritt (iii) des Homogenisierens des in (ii) erhaltenen Gemischs, der eine oder die mehreren Zeolithe vom MFI-Strukturtyp auf eine Partikelgröße D₅₀ im Bereich von 0,01 bis 200 µm, weiter bevorzugt von 0,03 bis 150 µm, weiter bevorzugt von 0,05 bis 100 µm, weiter bevorzugt von 0,1 bis 50 µm, weiter bevorzugt von 0,3 bis 30 µm, weiter bevorzugt von 0,4 bis 20 µm, noch weiter bevorzugt von 0,5 bis 15 µm gebracht werden.

Gemäß der vorliegenden Erfindung wird im bevorzugten Verfahren zur Herstellung des Katalysators ein Schritt des Trocknens nach Schritt (v) optional durchgeführt. Bezüglich der Art und Weise, durch welche die optionale Trocknung erzielt wird, besteht prinzipiell keinerlei Einschränkung, so dass die Trocknung bei jeder geeigneten Temperatur sowie in jeder geeigneten Atmosphäre durchgeführt werden kann. Somit kann die optionale Trocknung unter Schutzgasatmosphäre oder in Luft erfolgen, wobei die optionale Trocknung bevorzugt in Luft erfolgt. Bezüglich der Temperatur, bei welcher die Trocknung erfolgt, kann beispielsweise eine Temperatur gewählt werden, welche im Bereich von 50 bis 220 °C liegt. Gemäß vorliegender Erfindung erfolgt die optionale Trocknung nach Schritt (v) bei einer Temperatur im Bereich von 70 bis 180 °C, weiter bevorzugt von 80 bis 150 °C, weiter bevorzugt von 90 bis 130 °C und weiter bevorzugt im Bereich von 100 bis 120 °C. Gemäß besonders bevorzugten Ausführungsformen des erfindungsgemäßen Verfahrens erfolgt die Trocknung nach Schritt (v) bei einer Temperatur im Bereich von 105 bis 115 °C. Bezüglich der Dauer des einen oder der mehreren optionalen Schritte des Trocknens, insbesondere gemäß besonderen und bevorzugten Ausführungsformen des erfindungsgemäßen Verfahrens besteht keine besondere Einschränkung, sofern eine für die weiteren Verfahrensschritte geeignete Trocknung erzielt werden kann, beispielsweise nach einem Trocknungsschritt mit einer Dauer von 0,1 bis 20 Stunden. Gemäß besonderen Ausführungsformen des erfindungsgemäßen Verfahrens wird das optionale Trocknen für eine Dauer von 0,3 bis 10 h durchgeführt, weiter bevorzugt von 0,5 bis 5 h, weiter bevorzugt von 0,8 bis 2 h und noch weiter bevorzugt von 0,9 bis 1,5 h.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen das Trocknen in (v) bei einer Temperatur im Bereich von 50 bis 220°C, bevorzugt von 70 bis 180°C, weiter bevorzugt von 80 bis 150°C, weiter bevorzugt von 90 bis 130°C, weiter bevorzugt von 100 bis 120°C, und weiter bevorzugt von 105 bis 115°C erfolgt.

Hinsichtlich des optionalen Kalzinierens gemäß vorliegender Erfindung gilt prinzipiell das Gleiche wie bezüglich des optionalen Schritts des Trocknens, so dass auch hier keinerlei besondere Einschränkung existiert, weder hinsichtlich der Temperatur, noch hinsichtlich der Atmosphäre, in welcher die Kalzinierung durchgeführt wird, und schließlich auch nicht hinsichtlich der Dauer einer Kalzinierung gemäß den besonderen und bevorzugten Ausführungsformen der vorliegenden Erfindung, solange es sich bei dem Produkt der Kalzinierung um ein Zwischenprodukt handelt, welches sich dazu eignet, in den weiteren Schritten des erfindungsgemäßen Verfahrens zu einem Katalysator gemäß vorliegender Erfindung verarbeitet werden zu können. Somit kann beispielsweise hinsichtlich der Temperatur des optionalen Kalzinierens in Schritt (vi) eine Temperatur im Bereich von 300 bis 850 °C gewählt werden, wobei bevorzugt eine Temperatur im Bereich von 400 bis 750 °C, weiter bevorzugt von 450 bis 700 °C, weiter bevorzugt von 500 bis 650 °C und noch weiter bevorzugt von 530 bis 600 °C gewählt wird. Gemäß noch weiter bevorzugter Ausführungsformen der vorliegenden Erfindung wird die Kalzinierung in dem optionalen Schritt (vi) bei einer Temperatur von 540 bis 560 °C durchgeführt. Hinsichtlich der Atmosphäre, in welcher die optionale Kalzinierung gemäß einem oder mehreren der zuvor genannten Schritte des erfindungsgemäßen Verfahrens durchgeführt wird, so kann es sich hierbei sowohl um eine inerte Atmosphäre als auch um Luft handeln, wobei die optionale Kalzinierung in Schritt (vi) bevorzugt in Luft durchgeführt wird. Schließlich besteht auch keinerlei Einschränkung bezüglich der Dauer des Kalzinierungsschrittes in dem optionalen Schritt (vi). Somit kann die Dauer der Kalzinierung im optionalen Kalzinierungsschritte in (vi) beispielsweise 0,5 bis 20 Stunden betragen, wobei eine Dauer von 1 bis 15 h bevorzugt wird, weiter bevorzugt von 2 bis 10 h, weiter bevorzugt von 3 bis 7 h und eine Dauer von 4 bis 5 h besonders bevorzugt ist.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen das Kalzinieren in (vi) bei einer Temperatur im Bereich von 300 bis 850°C, bevorzugt von 400 bis 750°C, weiter bevorzugt von 450 bis 700°C, weiter bevorzugt von 500 bis 650°C, weiter bevorzugt von 530 bis 600°C, und weiter bevorzugt von 540 bis 560°C erfolgt.

Gemäß Schritt (ii) des bevorzugten Verfahrens zur Herstellung des Katalysators werden der eine oder die mehreren Zeolithe vom MFI-Strukturtyp zunächst mit einem oder mehreren Lösungsmittel vermischt. Gemäß vorliegender Erfindung besteht keinerlei Einschränkung in Schritt (ii) was die Art und/oder Anzahl der hierfür verwendeten Lösungsmittel betrifft. Somit kann prinzipiell jedes geeignete Lösungsmittel oder Lösungsmittelgemisch in Schritt (ii) verwendet werden, sofern dieses geeignet ist, ein Homogenisieren in Schritt (iii) sowie das Beschichten in Schritt (iv) zu ermöglichen. So kann beispielsweise in Schritt (ii) ein oder mehrere Lösungsmittel, ausgewählt aus der Gruppe bestehend aus Alkoholen, Wasser, Mischungen von zwei oder mehr Alkoholen und Mischungen von Wasser und einem oder mehreren Alkoholen, verwendet werden. Gemäß bevorzugten Ausführungsformen der vorliegenden Erfindung werden das in (ii) verwendete eine oder die mehreren Lösungsmittel ausgewählt aus der Gruppe bestehend aus (C₁-C₆)-Alkoholen, Wasser, Mischungen von zwei oder mehreren (C₁-C₆)-Alkoholen und Mischungen von Wasser und einem oder mehreren (C₁-C₆)-Alkoholen, wobei das eine oder die mehreren Lösungsmittel weiter bevorzugt aus der Gruppe bestehend aus (C₁-C₄)-Alkoholen, Wasser, Mischungen von zwei oder mehreren (C₁-C₄)-Alkoholen und Mischungen von Wasser und einem oder mehreren (C₁-C₄)-Alkoholen sind. Gemäß weiter bevorzugten Ausführungsformen werden das eine oder die mehreren Lösungsmittel in Schritt (ii) ausgewählt aus der Gruppe bestehend aus Methanol, Ethanol, n-Propanol, Isopropanol, Wasser und Mischungen von zwei oder mehr davon, weiter bevorzugt aus der Gruppe bestehend aus Methanol, Ethanol, Wasser und Mischungen von zwei oder mehr davon, wobei noch weiter bevorzugt das Lösungsmittel Wasser ist, bevorzugt destilliertes Wasser.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen das in (ii) hergestellte Gemisch ein oder mehrere Lösungsmittel enthält, ausgewählt aus der Gruppe bestehend aus Alkoholen, Wasser, Mischungen von zwei oder mehreren Alkoholen, und Mischungen von Wasser und einem oder mehreren Alkoholen, bevorzugt aus der Gruppe bestehend aus (C₁-C₆) Alkoholen, Wasser, Mischungen von zwei oder mehreren (C₁-C₆) Alkoholen, und Mischungen von Wasser und einem oder mehreren (C₁-C₆) Alkoholen, weiter bevorzugt (C₁-C₄) Alkoholen, Wasser, Mischungen von zwei oder mehreren (C₁-C₄) Alkoholen, und Mischungen von Wasser und einem oder mehreren (C₁-C₄) Alkoholen, weiter bevorzugt bestehend aus Methanol, Ethanol, n-Propanol, Isopropanol, Wasser und Mischungen von zwei oder mehr davon, weiter bevorzugt bestehend aus Methanol, Ethanol, Wasser und Mischungen von zwei oder mehr davon, wobei weiter bevorzugt das Lösungsmittel Wasser, bevorzugt destilliertes Wasser ist.

Hinsichtlich der Feststoffkonzentration des in (ii) bereitgestellten Gemischs bestehen gemäß vorliegender Erfindung keinerlei besondere Einschränkungen, vorausgesetzt dass ein Homogenisieren des Gemischs gemäß Schritt (iii) sowie die Verwendung des in (vi) erhaltenen homogenisierten Gemischs für das Beschichten in (iv) möglich ist. Somit kann die Feststoffkonzentration des in (ii) bereitgestellten Gemischs beispielsweise im Bereich von 10-75 Gew.-% liegen, wobei die Feststoffkonzentration gemäß vorliegender Erfindung bevorzugt im Bereich von 15-65 Gew.-% und weiter bevorzugt im Bereich von 20-60 Gew.-% und weiter bevorzugt im Bereich von 25-55 Gew.-% und weiter bevorzugt im Bereich von 30-50 Gew.-% liegt. Gemäß besonders bevorzugten Ausführungsformen des erfindungsgemäßen Verfahrens zur Herstellung eines Katalysators liegt die Feststoffkonzentration des in (v) bereitgestellten Gemischs im Bereich von 35-45 Gew.-%.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen die Feststoffkonzentration des in (ii) hergestellten Gemischs im Bereich von 10 bis 75 Gew.-% liegt, bevorzugt von 15 bis 65 Gew.-%, weiter bevorzugt von 20 bis 60 Gew.-%, weiter bevorzugt von 25 bis 55 Gew.-%, weiter bevorzugt von 30 bis 50 Gew.-%, und weiter bevorzugt von 35 bis 45 Gew.-% liegt.

Auch hinsichtlich des Homogenisierens in Schritt (iii) besteht gemäß vorliegender Erfindung keinerlei besondere Einschränkung, so dass jede erdenkliche Verfahrensweise gewählt werden kann, um ein homogenes Gemisch des in Schritt (ii) hergestellten Gemischs zu erzeugen, wobei hierfür beispielsweise ein oder mehrere Verfahren verwendet werden können, ausgewählt aus der Gruppe bestehend aus Rühren, Kneten, Schütteln, Vibration oder Kombination aus zwei oder mehr davon. Gemäß vorliegender Erfindung wird das in Schritt (ii) hergestellte Gemisch bevorzugt durch Rühren und/oder durch Vibration in Schritt (iii) homogenisiert, wobei weiter bevorzugt die Homogenisierung in Schritt (iii) durch Vibration erfolgt, bevorzugt durch Ultraschall wie beispielsweise durch Verwendung eines Ultraschallbads in welche das zu homogenisierende Gemisch gebracht wird.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen das Homogenisieren in (iii) durch Rühren, Kneten, Schütteln, Vibration oder Kombinationen aus zwei oder mehr davon, bevorzugt durch Rühren und/oder Vibration, weiter bevorzugt durch Vibration, und weiter bevorzugt durch Ultraschall erfolgt.

Bezüglich des Beschichtens des Trägersubstrats in Schritt (iv) des erfindungsgemäßen Verfahrens besteht prinzipiell keinerlei Einschränkung hinsichtlich seiner Durchführung, vorausgesetzt eine entsprechende Schicht wird zumindest teilweise auf das Trägersubstrat hierdurch geformt. Somit kann jede geeignete Form des Beschichtens bzw. der Schichtbildung im erfindungsgemäßen Verfahren zur Herstellung des erfindungsgemäßen Katalysators angewandt werden, wobei das Beschichten in Schritt (iv) bevorzugt durch Sprühbeschichten und/oder Waschbeschichten erfolgt. Gemäß besonders bevorzugten Ausführungsformen des erfindungsgemäßen Verfahrens erfolgt das Beschichten in Schritt (iv) durch Waschbeschichten, wobei das Waschbeschichten bevorzugt durch Tauchbeschichten erfolgt. Ein solches bevorzugtes Tauchbeschichten erfolgt beispielsweise durch ein oder mehrmaliges Eintauchen des Trägersubstrats in das in Schritt (ii) hergestellte und in Schritt (iii) homogenisierte Gemisch, wobei gemäß vorliegender Erfindung dem Tauchbeschichten bevorzugt eine Behandlung zur Entfernung von überschüssigem Gemisch von dem Trägersubstrat folgt. Bei bevorzugten Ausführungsformen des Tauchbeschichtens, bei welchen das Substrat mehrmals in das in Schritt (ii) hergestellte und in Schritt (iii) homogenisierte Gemisch eingetaucht wird, kann die weiter bevorzugte Behandlung zur Entfernung von überschüssigem Gemisch prinzipiell nach dem mehrmaligen Tauchen und/oder zwischen zwei oder mehr Tauchschritten erfolgen, wobei bevorzugt nach jedem Schritt des Eintauchens überschüssiges Gemisch durch eine geeignete Behandlung vom beschichteten Trägersubstrat entfernt wird. Besonders bevorzugt wird jedoch gemäß der vorliegenden Erfindung ein Schritt des Eintauchens in das in Schritt (ii) hergestellte und in Schritt (iii) homogenisierte Gemisch durchgeführt, gefolgt von einer entsprechenden Behandlung zur Entfernung von überschüssigem Gemisch. Was die besonders bevorzugte Entfernung von überschüssigem Gemisch gemäß den besonderen Ausführungsformen des vorliegenden Verfahrens betrifft, bei welchen ein Tauchbeschichten in Schritt (iv) durchgeführt wird, besteht prinzipiell keinerlei Einschränkung hinsichtlich der Art und Weise, in welcher überschüssiges Gemisch entfernt wird. Somit kann eine Entfernung beispielsweise durch geeignetes Abhängen und/oder Stehenlassen des beschichteten Trägersubstrats und/oder direkt oder indirekt durch mechanische oder anderweitige Einwirkung erreicht werden, wie beispielsweise durch mechanisches Abstreifen und/oder durch Entfernen mit einem geeigneten Gasgebläse und/oder durch geeignete Anwendung von Zentripetalkräften wie beispielsweise durch auf geeignete Weise gerichtete Schleuderkräfte. Nach vorliegender Erfindung ist es jedoch besonders bevorzugt, die Entfernung von überschüssigem Gemisch durch ein Gasgebläse zu entfernen, besonders bevorzugt mithilfe von Druckluft durch geeignetes Ausblasen des überschüssigen Gemischs.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen das Beschichten in (iv) durch Sprühbeschichten und/oder Waschbeschichten, bevorzugt durch Waschbeschichten erfolgt, wobei das Waschbeschichten bevorzugt durch Tauchbeschichten erfolgt, welche bevorzugt von einer Behandlung zur Entfernung von überschüssigem Gemisch gefolgt wird, wobei die Entfernung von überschüssigem Gemisch bevorzugt zumindest teilweise mit Druckluft erfolgt.

Gemäß dem erfindungsgemäßen Verfahren besteht nach der vorliegenden Erfindung prinzipiell die Möglichkeit, das Trägersubstrat mit mehreren Schichten der gleichen und/oder unterschiedlicher Zusammensetzung insbesondere hinsichtlich des einen oder der mehreren Zeolithe vom MFI-Strukturtyp zu versehen. Somit werden Ausführungsformen des erfindungsgemäßen Verfahrens zur Herstellung eines Katalysators gemäß vorliegender Erfindung bevorzugt, in welchen Schritt (iv) ein- oder mehrmals wiederholt wird, wobei bevorzugt Schritt (v) und/oder Schritt (vi) und bevorzugt sowohl Schritt (v) als auch Schritt (vi) zwischen den Wiederholungen ausgeführt werden. Bei solchen bevorzugten Ausführungsformen des erfindungsgemäßen Verfahrens, in welchen zwei oder mehr Schichten unterschiedlicher Zusammensetzung insbesondere hinsichtlich des einen oder der mehreren Zeolithe auf das Trägersubstrat aufgebracht werden, werden auch die Schritte (ii) und (iii) entsprechend wiederholt bei der Herstellung der unterschiedlichen Zusammensetzungen des Gemischs in Schritt (ii), wobei sich dies nicht nur auf die chemische Zusammensetzung, sondern auch auf weitere Eigenschaften des Gemischs beziehen kann, wie beispielsweise die durchschnittliche Partikelgröße des einen oder der mehreren Zeolithe vom MFI-Strukturtyp. Gemäß besonders bevorzugten Ausführungsformen des erfindungsgemäßen Verfahrens werden die Schritte (iv) und (v) und/oder (vi) bevorzugt die Schritte (iv)-(vi) ein- oder mehrmals wiederholt, um ein mehrmaliges Beschichten des Trägersubstrats mit einem in Schritt (ii) hergestellten und in Schritt (iii) homogenisierten Gemischs zu erreichen.

Bezüglich der Anzahl der Wiederholungen, welche gemäß den bevorzugten Ausführungsformen des erfindungsgemäßen Verfahrens zur Herstellung eines Katalysators gemäß vorliegender Erfindung besteht prinzipiell keine Einschränkung, wobei die Schritte bei den Wiederholungen gemäß den besonderen und bevorzugten Ausführungsformen des erfindungsgemäßen Verfahrens bevorzugt einmal bis fünfmal wiederholt werden, weiter bevorzugt einmal bis viermal, weiter bevorzugt einmal bis dreimal und weiter bevorzugt einmal oder zweimal wiederholt werden.

Somit werden gemäß der vorliegenden Erfindung Ausführungsformen des Verfahrens zur Herstellung des Katalysators, und insbesondere des Katalysators gemäß einer der besonderen oder bevorzugten Ausführungsformen dieser, bevorzugt, gemäß welchen Schritt (iv) ein oder mehrmals wiederholt wird, bevorzugt die Schritte (iv) und (v), weiter bevorzugt die Schritte (iv) bis (vi), wobei die Schritte bevorzugt einmal bis fünfmal, weiter bevorzugt einmal bis viermal, weiter bevorzugt einmal bis dreimal, und weiter bevorzugt einmal oder zweimal wiederholt werden.

Gemäß den besonders bevorzugten Ausführungsformen der vorliegenden Erfindung nach welchen der eine oder die mehreren Zeolithe vom MFI-Strukturtyp im Katalysator in deren H-Form jeweils vorliegen können diese nach entsprechend bevorzugten Ausführungsformen des Verfahrens zur Herstellung des Katalysators entweder in der H-Form in Schritt (i) bereitgestellt werden und/oder während des Verfahrens durch geeignete Behandlung und insbesondere durch lonenaustausch in die H-Form gebracht werden. Gemäß den bevorzugten Ausführungsformen des Verfahrens zur Herstellung des Katalysators nach welchen der eine oder die mehreren Zeolithe während der Herstellung in die H-Form gebracht werden, bestehen prinzipiell keine besonderen Einschränkungen hinsichtlich der Art und Weise auf welcher dies durchgeführt wird, wobei die Überführung des einen oder der mehreren Zeolithe bevorzugt durch lonenaustausch erfolgt. Die Überführung des einen oder der mehreren Zeolithe in die H-Form kann somit auch an jeder Stelle des Verfahrens erfolgen, wobei diese bevorzugt nach dem Herstellen des Gemischs in (ii) oder nach dem Beschichten und optionalen Trocknen und/oder Kalzinieren durchgeführt wird, bevorzugt nach dem Trocknen des beschichteten Trägersubstrats in (v) und besonders bevorzugt nach dem Kalzinieren des beschichteten Trägersubstrats in (vi), wobei die Überführung in die H-Form bevorzugt an dem getrockneten und kalzinierten beschichteten Trägersubstrat erfolgt.

Hinsichtlich der bevorzugten Ausführungsformen des Verfahrens zur Herstellung des Katalysators nach welchen die Überführung des einen oder der mehreren Zeolithe vom MFI Strukturtyp in die H-Form über einen oder mehrere Schritte eines lonenaustauschs erfolgt besteht wiederum keine besonderen Einschränkungen hinsichtlich der Art und Weise wie diese durchgeführt werden, vorausgesetzt zumindest ein Teil der Gegenionen zum Zeolithgerüst durch H⁺-Ionen ausgetuascht werden können. Nach bevorzugten Ausführungsformen wird zwecks lonenaustausch der eine oder die mehreren Zeolithe mit einer Lösung einer protonierten leichtflüchtigen Base in Kontakt gebracht, bevorzugt eines protonierten leichtflüchtigen Amins, besonders bevorzugt mit einer Ammoniumsalz-Lösung, oder alternativ hierzu mit einer Säure und bevorzugt mit einer wässerigen Säure-Lösung in Kontakt gebracht, bevorzugt mit einer wässerigen Lösung einer Mineralsäure. Hinsichtlich der Ammoniumsalze welche bevorzugt verwendet werden besteht keine generelle Einschränkung, vorausgesetzt der Austausch zumindest eines Teils der in dem einen oder den mehreren Zeolithen enthaltenen Gegenionen durch Ammonium erfolgen kann. So können zu diesem Zweck beispielsweise ein oder mehrere Ammoniumsalze verwendet werden ausgewählt aus der Gruppe bestehend aus NH₄NO₃, NH₄Cl, (NH₄)₂SO₄ sowie Mischungen aus zwei oder mehr davon. Gleiches gilt entsprechend hinsichtlich der Säuren und insbesondere der Mineralsäuren welche zwecks lonenaustausch eingesetzt werden können, vorausgesetzt der Austausch zumindest eines Teils der in dem einen oder den mehreren Zeolithen enthaltenen Gegenionen durch H⁺ erfolgen kann. Somit können beispielsweise Lösungen der Mineralsäuren HNO₃, HCl, H₂SO₄ sowie auch Mischungen von zwei oder mehr davon für den lonenaustausch eingesetzt werden. Hinsichtlich der Konzentration der für den bevorzugten lonenaustausch verwendeten Lösungen an protonierten leichtflüchtigen Basen oder an Säuren besteht keinerlei besondere Einschränkung, vorausgesetzt zumindest ein Teil der Gegenionen des Zeolithgerüsts kann ausgetauscht werden, und im Falle der Verwendung einer oder mehreren Säuren, dass der pH-Wert der Lösung zu keiner wesentlichen Auflösung des Zeolithgerüsts führt. Somit können beispielsweise Lösungen der Salze oder der Säuren verwendet werden mit einer Konzentration von 1 bis 50 Gew.-%, wobei bevorzugt Konzentrationen von 5 bis 30 Gew.-%, und besonders bevorzugt von 10 bis 25 Gew.% für den lonenaustausch verwendet werden. Gleiches gilt entsprechend hinsichtlich des Gewichtsverhältnisses von Salz- oder Säurelösung zu dem einen oder den mehreren Zeolithen welche ionenausgetauscht werden. Somit kann das Gewichtsverhältnis der verwendeten Lösung für den lonenaustausch zu dem einen oder den mehreren Zeolithen beispielweise im Bereich von 1 bis 20 liegen, wobei das Gewichtsverhältnis bevorzugt im Bereich von 2 bis 10, und weiter bevorzugt im Bereich von 4 bis 7 liegt.

Der lonenaustausch kann hierbei grundsätzlich vor Bereitstellen des einen oder der mehreren Zeolithe in Schritt (i) erfolgen, oder nach einem oder mehreren der Schritte des bevorzugten Verfahrens zur Herstellung des Katalysators, wobei ein lonenaustausch bevorzugt vor dem Bereitstellen in Schritt (i) und/oder nach dem Beschichten und optionalen Trocknen und/oder Kalzinieren durchgeführt wird, bevorzugt nach dem Trocknen des beschichteten Trägersubstrats in (v) und besonders bevorzugt nach dem Kalzinieren des beschichteten Trägersubstrats in (vi). Gemäß den bevorzugten Ausführungsformen der Herstellung des im erfindungsgemäßen Verfahren verwendeten Katalysators in welchen ein Schritt des lonenaustauschs mit einer protonierten leichtflüchtigen Base, und bevorzugt mit einem protonierten leichtflüchtigen Amin, besonders bevorzugt mit Ammonium, nach dem Kalzinieren in (vi) durchgeführt wird, ist es weiter bevorzugt dass nach dem lonenausstausch und nach einem optionalen Waschritt und/oder nach einem optionalen Trocknungsschritt ein weiterer Schritt des Kalzinierens durchgeführt wird um die leichtflüchtige Base und besonders bevorzugt Ammoniak vollständig aus dem ionenausgetauschten Zeolith zu entfernen.

### BEISPIELE

### Vergleichsbeispiel 1: Herstellen eines Extrudats enthaltend ZSM-5

380 g H-ZSM-5 (ZEO-cat PZ2-100 H von der Fa. Zeochem) mit Si/Al = 50 wurden mit 329 g Pseudoböhmit (Pural SB; Sasol) vermischt, mit 10 g Ameisensäure in 50 ml Wasser versetzt und mit 300 ml Wasser im Kneter zu einer homogenen Masse verarbeitet. Die Einwaagen wurden dabei so gewählt, dass das Zeolith/Binder-Verhältnis im kalzinierten Extrudate 60:40 entspricht. Diese Knetmasse wurde mit Hilfe einer Strangpresse mit ca. 100 bar durch eine 2,5 mm Matrize geschoben. Die Stränge wurden anschießend 16 h bei 120°C im Trockenschrank getrocknet und (nach 4 h Aufheizzeit) 4 h bei 500°C im Muffelofen kalziniert. Danach wurden die Stränge in einer Siebmaschine mit 2 Stahlkugeln (Durchmesser ca. 2 cm, 258 g/Kugel) zu 1,6-2,0 mm Splitt verarbeitet.

### Beispiel 1: Herstellen eines mit ZSM-5 beschichteten Trägers

Eine wässrige Suspension mit einer Feststoffkonzentration von 40 Gew.-% an H-ZSM-5 Zeolith (ZEO-cat PZ2-100 H von der Fa. Zeochem) mit Si/Al = 50 wurde hergestellt und im Ultraschallbad homogenisiert. Zylindrische Wabenkörperstücke aus Cordierit (900 cpsi, Durchmesser 0.9 cm, Länge = 11 cm) wurden in diese Suspension eingetaucht und anschließend mit Druckluft ausgeblasen. Die beschichteten Träger wurden dann für 1 h bei 110°C getrocknet und anschließend für 3 h bei 550°C kalziniert. Der Beschichtungsschritt wurde wiederholt bis eine Beladung von 0,5 g Zeolith pro Wabenkörperstück (0,071 g/cm³) erreicht wurde.

### Beispiel 2: Methanol-zu-Olefin-Verfahren mit vorgelagerter Umwandlung von Methanol zu Dimethylether

2 g des gemäß Vergleichsbeispiel 1 hergestellten Katalysators wurden mit 24 g Siliziumcarbid vermischt und in einem kontinuierlich betriebenen, elektrisch beheizten Rohrreaktor eingebaut, so dass die Schüttung im Reaktor eine Länge von 30 cm und einen Durchmesser von 12 mm besitzt. Für die Versuche unter Verwendung des gemäß Beispiel 1 hergestellten Katalysators wurden zwei der beschichteten Wabenkörper in den Reaktor eingebaut und an der Rohrwand mit Glasfaser-Schnur abgedichtet.

Vor dem Testreaktor wurde Methanoldampf erzeugt zu einem Gasstrom enthaltend 75 Vol.-% Methanol und 25 Vol.-% N₂ welcher durch einen mit 34 mL Aluminiumoxid-Splitt beladenen Vorreaktor bei 275°C und einem (absoluten) Druck von 1-2 bar zu Dimethylether umgesetzt wurde. Der Dimethylether enthaltende Strom wurde dann in den Rohrreaktor geleitet, und dort bei einer Temperatur von 450 bis 500°C, einer Belastung (WHSV = weight hourly space velocity, wird als Verhältnis von Oxygenat-Eduktstrom in kg/h zur Menge von Zeolith im Reaktor in kg errechnet) von 7 bzw. 10 h⁻¹ bezogen auf Methanol sowie bei einem (absoluten) Druck von 1 bis 2 bar umgesetzt, wobei die Reaktionsparameter während der gesamten Laufzeit gehalten wurden. Nach dem Rohrreaktor wurde das gasförmige Produkt-Gemisch on-line chromatographisch analysiert.

Nach erfolgter Durchführung eines Zyklus mit dem Katalysator gemäß Beispiel 1 wurde der Katalysator ausgebaut und in einem Muffelofen für 5h bei 500°C in Luftatmosphäre kalziniert, wobei das Koks fast vollständig verbrannt wurde. Der regenerierte Katalysator wurde anschließend erneut in den Testreaktor eingesetzt unter den gleichen Bedingungen wie der frische Katalysator aus Beispiel 1.

Die im MTO-Verfahren für die Katalysatoren gemäß Vergleichsbeispiel 1 sowie gemäß Beispiel 1 (vor und nach der Regenierung des Katalysators) erzielten Ergebnisse hinsichtlich der Selektivitäten sind in Tablle 1 dargestellt, wobei diese die Durchschnittsselektivitäten während der Laufzeit des Katalysators wiedergeben, bei welcher der Umsatz von Methanol 95% oder mehr betrug.

**Tabelle 1: Durchschnittsselektivitäten eines Zyklus (Methanol-Umsatz von >95%).**

| | Vergleichsbeispiel 1 | Beispiel 1 | Beispiel 1 nach Regenerierung |
|---|---|---|---|
| Standzeit [h] | 33 | 53 | 68 |
| WHSV [h⁻¹] | 10 | 7 | 7 |
| MeOH-Last pro Zyklus [kg_{MeOH} · kg-_{Zeolith}⁻¹] | 330 | 371 | 476 |
| Selektivität [%]: | | | |
| Ethylen | 9 | 8 | 8 |
| Propylen | 24 | 19 | 25 |
| Butylen | 15 | 17 | 20 |
| C₄-Paraffine | 10 | 12 | 9 |
| C₅₊ (Gemisch) | 16 | 18 | 20 |
| Aromaten | 19 | 18 | 13 |
| C₁-C₃ Paraffine | 7 | 8 | 5 |

Wie den Werten in Tabelle 1 entnommen werden kann, wurde überraschenderweise festgestellt, dass die spezielle Verwendung eines Zeoliths welcher auf einem Trägersubstrat aufgebracht wurde in einem MTO-Verfahren mit einer Vorreaktion von Methanol zu Dimethylether eine überraschend lange Standzeit bzw. eine unerwartet hohe Methanol-Last pro Zyklus des Katalysators ermöglicht, bei welchem ein Umsatz an Methanol von über 95% aufrechterhalten werden kann. Um so überraschender ist der Umstand, dass die Regenerierung des Katalysators durch Kalzinierung zu einer weiteren erheblichen Steigerung der Standzeit führte (siehe Beispiel 1 nach Regenerierung in Tabelle 1). Darüber hinaus zeigen die Ergebnisse der Umsetzung für den regenerierten Katalysator, dass dieser auch gegenüber dem frischen Katalysator eine weitere Steigerung der Selektivität zu Butylen aufweist, sowie auch eine Selektivität zu Propylen zeigt welche höher ist als die für das Vergleichsbeispiel erzielte Selektivität. Somit wird durch die vorliegende Erfindung ein Verfahren für die Umwandlung von Ethern zu Olefinen bereitgestellt welcher, wie anhand der Testergebnisse im MTO-Verfahren unter Verwendung des Katalysators gemäß Beispiel 1 gezeigt werden konnte, wesentlich längere Standzeiten ermöglicht gegenüber einem solchen Verfahren welches ein Katalysator in Form eines Extrudats verwendet (siehe Ergebnisse mit dem Katalysator aus Vergleichsbeispiel 1). Darüberhinaus erzielt das Verfahren überraschender Weise eine zusätzliche Steigerung hin zu längeren Standzeiten als auch unerwartet höhere C₃- und C₄-Selektivitäten gegenüber dem Vergleichsbeispiel nach der Regenerierung des Katalysators durch Kalzinierung.

### Zitierte Dokumente des Standes der Technik

- Antia et al. in Ind. Eng. Chem. Res. 1995, 34, Seiten 140-147
- US 4,692,423
- Ivanova et al. in J. Phys. Chem. C 2007, 111, Seiten 4368-4374
- Patcas, F. C. in Journal of Catalysis 2005, 231, Seiten 194-200
- WO 98/29519 A1
- WO 94/25151 A1
- Hammon et al. in Applied Catalysis 1988, 37, Seiten 155-174
- Li et al. in Catal. Lett. 2009, 129, Seiten 408-415
- US 4,049,573
- Goryainova et al. in Petroleum Chemistry 2011, Bd. 51, Nr. 3, S. 169-173
- JP 2007 137840
- US 2002/038775 A1
- EP 2 446 964 A1

## Patentansprüche

1. Verfahren zur Umwandlung von Oxygenaten zu Olefinen umfassend
(1) Bereitstellen eines Gasstroms enthaltend ein oder mehrere Ether;
(2) Inkontaktbringen des in (1) bereitgestellten Gasstroms mit einem Katalysator, wobei das Inkontaktbringen bei einer Temperatur im Bereich von 430 bis 520°C erfolgt;
(3) Kalzinierung des Katalysators zur Regenerierung wobei die Kalzinierung bei einer Temperatur im Bereich von 450 bis 550°C in Luftatmosphäre erfolgt;
(4) Bereitstellen eines Gasstroms enthaltend ein oder mehrere Ether;
(5) Inkontaktbringen des in (4) bereitgestellten Gasstroms mit dem regenerierten Katalysator,
wobei der Katalysator
- ein Trägersubstrat und
- eine auf das Substrat aufgebrachte Schicht umfasst,
wobei die Schicht ein oder mehrere Zeolithe vom MFI -Strukturtyp enthält, und wobei das Trägersubstrat keramische und/oder metallische Stoffe enthält.

2. Verfahren nach Anspruch 1, wobei die Form des Trägersubstrats ausgewählt ist aus der Gruppe bestehend aus Granulaten, Pellets, Netzen, Ringen, Kugeln, Zylinder, Hohlzylinder, Monolithen und Mischungen und/oder Kombinationen von zwei oder mehr davon.

3. Verfahren nach Anspruch 2, wobei der ein oder die mehreren Monolithe ausgewählt sind aus der Gruppe bestehend aus Waben, Geflechte, Schäume, und Kombinationen von zwei oder mehr davon.

4. Verfahren nach einem der Ansprüche 1 bis 3, wobei der Katalysator den einen oder die mehreren Zeolithe vom MFI -Strukturtyp in einer Gesamtbeladung von 0,005 bis 1 g/cm³ bezogen auf das Volumen des beschichteten Trägersubstrats enthält.

5. Verfahren nach einem der Ansprüche 1 bis 4, wobei der Gasstrom gemäß (1) ein oder mehrere Di-(C₁-C₃)alkylether enthält.

6. Verfahren nach einem der Ansprüche 1 bis 5, wobei der Gehalt an Ethern in dem Gasstrom gemäß (1) im Bereich von 30 bis 100 Vol.-% bezogen auf das Gesamtvolumen liegt.

7. Verfahren nach einem der Ansprüche 1 bis 6, wobei der Gasstrom gemäß (1) aus einer Vorstufe der Dehydratisierung von einem oder mehreren aliphatischen Alkoholen erhältlich ist.

8. Verfahren nach einem der Ansprüche 1 bis 7, wobei der Gehalt an Wasser in dem Gasstrom gemäß (1) im Bereich von 5 bis 60 Vol.-% bezogen auf das Gesamtvolumen liegt.

9. Verfahren nach einem der Ansprüche 1 bis 8, wobei das Inkontaktbringen gemäß (2) bei einem Druck im Bereich von 0,1 bis 10 bar erfolgt.

10. Verfahren nach einem der Ansprüche 1 bis 9, wobei das Verfahren ein kontinuierliches Verfahren ist.

11. Verfahren nach Anspruch 10, bei dem die Raumgeschwindigkeit beim Inkontaktbringen gemäß (2) im Bereich von 0,5 bis 50 h⁻¹ liegt.

12. Verfahren nach Anspruch 11, bei dem die Standzeit des Katalysators während der das kontinuierliche Verfahren ohne Unterbrechung durchgeführt wird im Bereich von 15 bis 200 h liegt.

13. Verfahren nach Anspruch 1, wobei die Kalzinierung in (3) bei einer Temperatur im Bereich von 200 bis 1.100°C durchgeführt wird.

14. Verfahren nach Anspruch 1 oder 13, wobei die Kalzinierung in (3) für eine Dauer von 0,25 bis 30 h durchgeführt wird.

15. Verfahren nach einem der Ansprüche 1, 13 oder 14, wobei die Schritte (3) bis (5) ein bis 1000 Mal wiederholt werden.

16. Verfahren nach einem der Ansprüche 1 bis 15, wobei der Katalysator erhältlich ist nach einem Verfahren umfassend
(i) Bereitstellen des Trägersubstrats und des einen oder der mehreren Zeolithe vom MFI -Strukturtyp;
(ii) Herstellen eines Gemischs, enthaltend den einen oder die mehreren Zeolithe vom MFI-Strukturtyp und ein oder mehrere Lösungsmittel;
(iii) Homogeniseren des in (ii) erhaltenen Gemischs;
(iv) Beschichten des Trägersubstrats mit dem in (iii) erhaltenen homogenisierten Gemisch;
(v) optionales Trocknen des in (iv) erhaltenen beschichteten Trägersubstrats;
(vi) optionales Kalzinieren des in (iv) oder (v) erhaltenen beschichteten Trägersubstrats.

17. Verfahren nach Anspruch 16, wobei das Trocknen in (v) bei einer Temperatur im Bereich von 50 bis 220°C erfolgt.

18. Verfahren nach Anspruch 16 oder 17, wobei das Kalzinieren in (vi) bei einer Temperatur im Bereich von 300 bis 850°C erfolgt.

19. Verfahren nach einem der Ansprüche 16 bis 18, wobei das in (ii) hergestellte Gemisch ein oder mehrere Lösungsmittel enthält, ausgewählt aus der Gruppe bestehend aus Alkoholen, Wasser, Mischungen von zwei oder mehreren Alkoholen, und Mischungen von Wasser und einem oder mehreren Alkoholen.

20. Verfahren nach einem der Ansprüche 16 bis 19, wobei die Feststoffkonzentration des in (ii) hergestellten Gemischs im Bereich von 10 bis 75 Gew.-% liegt.

21. Verfahren nach einem der Ansprüche 16 bis 20, wobei das Homogenisieren in (iii) durch Rühren, Kneten, Schütteln, Vibration oder Kombinationen aus zwei oder mehr davon erfolgt.

22. Verfahren nach einem der Ansprüche 16 bis 21, wobei das Beschichten in (iv) durch Sprühbeschichten und/oder Waschbeschichten erfolgt.

23. Verfahren nach einem der Ansprüche 16 bis 22, wobei Schritt (iv) ein oder mehrmals wiederholt wird.

## Claims

1. A process for converting oxygenates to olefins, comprising
(1) providing a gas stream comprising one or more ethers;
(2) contacting the gas stream provided in (1) with a catalyst, wherein the contacting is effected at a temperature in the range from 430 to 520°C;
(3) calcining the catalyst for regeneration, wherein the calcination is effected at a temperature in the range from 450 to 550°C in air atmosphere;
(4) providing a gas stream comprising one or more ethers;
(5) contacting the gas stream provided in (4) with the regenerated catalyst, the catalyst comprising
- a support substrate and
- a layer applied to the substrate, the layer comprising one or more zeolites of the MFI structure type, and the support substrate comprising ceramic and/or metallic substances.

2. The process according to claim 1, wherein the form of the support substrate is selected from the group consisting of granules, pellets, meshes, rings, spheres, cylinders, hollow cylinders, monoliths and mixtures and/or combinations of two or more thereof.

3. The process according to claim 2, wherein the one or more monoliths are selected from the group consisting of honeycombs, braids, foams and combinations of two or more thereof.

4. The process according to any of claims 1 to 3, wherein the catalyst comprises the one or more zeolites of the MFI structure type in a total loading of 0.005 to 1 g/cm³ based on the volume of the coated support substrate.

5. The process according to any of claims 1 to 4, wherein the gas stream according to (1) comprises one or more di(C₁-C₃)alkyl ethers.

6. The process according to any of claims 1 to 5, wherein the content of ethers in the gas stream according to (1) is in the range from 30 to 100% by volume based on the total volume.

7. The process according to any of claims 1 to 6, wherein the gas stream according to (1) is obtainable from a precursor of the dehydration of one or more aliphatic alcohols.

8. The process according to any of claims 1 to 7, wherein the water content in the gas stream according to (1) is in the range from 5 to 60% by volume based on the total volume.

9. The process according to any of claims 1 to 8, wherein the contacting according to (2) is effected at a pressure in the range from 0.1 to 10 bar.

10. The process according to any of claims 1 to 9, wherein the process is a continuous process.

11. The process according to claim 10, in which the space velocity in the contacting according to (2) is in the range from 0.5 to 50 h⁻¹.

12. The process according to claim 11, in which the service life of the catalyst during which the continuous process is performed without interruption is in the range from 15 to 200 h.

13. The process according to claim 1, wherein the calcination in (3) is performed at a temperature in the range from 200 to 1100°C.

14. The process according to claim 1 or 13, wherein the calcination in (3) is performed for a period of 0.25 to 30 h.

15. The process according to any of claims 1, 13 and 14 wherein steps (3) to (5) are repeated once to 1000 times.

16. The process according to any of claims 1 to 15, wherein the catalyst is obtainable by a process comprising
(i) providing the support substrate and the one or more zeolites of the MFI structure type;
(ii) preparing a mixture comprising the one or more zeolites of the MFI structure type and one or more solvents;
(iii) homogenizing the mixture obtained in (ii) ;
(iv) coating the support substrate with the homogenized mixture obtained in (iii);
(v) optionally drying the coated support substrate obtained in (iv);
(vi) optionally calcining the coated support substrate obtained in (iv) or (v).

17. The process according to claim 16, wherein the drying in (v) is effected at a temperature in the range from 50 to 220°C.

18. The process according to claim 16 or 17, wherein the calcining in (vi) is effected at a temperature in the range from 300 to 850°C.

19. The process according to any of claims 16 to 18, wherein the mixture prepared in (ii) comprises one or more solvents selected from the group consisting of alcohols, water, mixtures of two or more alcohols, and mixtures of water and one or more alcohols.

20. The process according to any of claims 16 to 19, wherein the solids concentration of the mixture prepared in (ii) is in the range from 10 to 75% by weight.

21. The process according to any of claims 16 to 20, wherein the homogenizing in (iii) is effected by stirring, kneading, agitating, vibrating or combinations of two or more thereof.

22. The process according to any of claims 16 to 21, wherein the coating in (iv) is effected by spray coating and/or wash coating.

23. The process according to any of claims 16 to 22, wherein step (iv) is repeated once or more than once.

## Revendications

1. Procédé de transformation de composés oxygénés en oléfines, comprenant :
(1) la préparation d'un courant gazeux contenant un ou plusieurs éthers ;
(2) la mise en contact du courant gazeux préparé en (1) avec un catalyseur, la mise en contact ayant lieu à une température dans la plage allant de 430 à 520 °C ;
(3) la calcination du catalyseur pour la régénération, la calcination ayant lieu à une température dans la plage allant de 450 à 550 °C dans l'atmosphère de l'air ;
(4) la préparation d'un courant gazeux contenant un ou plusieurs éthers ;
(5) la mise en contact du courant gazeux préparé en (4) avec le catalyseur régénéré,
le catalyseur comprenant
- un substrat support, et
- une couche appliquée sur le substrat,
la couche contenant une ou plusieurs zéolithes de type structural MFI, et
le substrat support contenant des matières céramiques et/ou métalliques.

2. Procédé selon la revendication 1, dans lequel la forme du substrat support est choisie dans le groupe constitué par les granulats, les pastilles, les filets, les anneaux, les billes, les cylindres, les cylindres creux, les monolithes et les mélanges et/ou combinaisons de deux ou plus d'entre eux.

3. Procédé selon la revendication 2, dans lequel le ou les monolithes sont choisis dans le groupe constitué par les nids d'abeilles, les tissus, les mousses et les combinaisons de deux ou plus d'entre eux.

4. Procédé selon l'une quelconque des revendications 1 à 3, dans lequel le catalyseur contient la ou les zéolithes de type structural MFI en une charge totale de 0,005 à 1 g/cm³ par rapport au volume du substrat support revêtu.

5. Procédé selon l'une quelconque des revendications 1 à 4, dans lequel le courant gazeux selon (1) contient un ou plusieurs éthers di-alkyliques en (C₁-C₃) .

6. Procédé selon l'une quelconque des revendications 1 à 5, dans lequel la teneur en éthers dans le courant gazeux selon (1) se situe dans la plage allant de 30 à 100 % en volume par rapport au volume total.

7. Procédé selon l'une quelconque des revendications 1 à 6, dans lequel le courant gazeux selon (1) peut être obtenu par une étape préliminaire de déshydratation d'un ou de plusieurs alcools aliphatiques.

8. Procédé selon l'une quelconque des revendications 1 à 7, dans lequel la teneur en eau dans le courant gazeux selon (1) se situe dans la plage allant de 5 à 60 % en volume, par rapport au volume total.

9. Procédé selon l'une quelconque des revendications 1 à 8, dans lequel la mise en contact selon (2) a lieu à une pression dans la plage allant de 0,1 à 10 bar.

10. Procédé selon l'une quelconque des revendications 1 à 9, dans lequel le procédé est un procédé continu.

11. Procédé selon la revendication 10, dans lequel la vitesse spatiale lors de la mise en contact selon (2) se situe dans la plage allant de 0,5 à 50 h⁻¹.

12. Procédé selon la revendication 11, dans lequel la durée d'utilisation du catalyseur pendant laquelle le procédé continu est réalisé sans interruption se situe dans la plage allant de 15 à 200 h.

13. Procédé selon la revendication 1, dans lequel la calcination en (3) est réalisée à une température dans la plage allant de 200 à 1 100 °C.

14. Procédé selon la revendication 1 ou 13, dans lequel la calcination en (3) est réalisée pendant une durée de 0,25 à 30 h.

15. Procédé selon l'une quelconque des revendications 1, 13 ou 14, dans lequel les étapes (3) à (5) sont répétées une à 1 000 fois.

16. Procédé selon l'une quelconque des revendications 1 à 15, dans lequel le catalyseur peut être obtenu par un procédé comprenant :
(i) la préparation du substrat support et de la ou des zéolithes de type structural MFI ;
(ii) la fabrication d'un mélange, contenant la ou les zéolithes de type structural MFI et un ou plusieurs solvants ;
(iii) l'homogénéisation du mélange obtenu en (ii) ;
(iv) le revêtement du substrat support avec le mélange homogénéisé obtenu en (iii) ;
(v) le séchage éventuel du substrat support revêtu obtenu en (iv) ;
(vi) la calcination éventuelle du substrat support revêtu obtenu en (iv) ou (v).

17. Procédé selon la revendication 16, dans lequel le séchage en (v) a lieu à une température dans la plage allant de 50 à 220 °C.

18. Procédé selon la revendication 16 ou 17, dans lequel la calcination en (vi) a lieu à une température dans la plage allant de 300 à 850 °C.

19. Procédé selon l'une quelconque des revendications 16 à 18, dans lequel le mélange fabriqué en (ii) contient un ou plusieurs solvants choisis dans le groupe constitué par les alcools, l'eau, les mélanges de deux alcools ou plus et les mélanges d'eau et d'un ou de plusieurs alcools.

20. Procédé selon l'une quelconque des revendications 16 à 19, dans lequel la concentration en solides du mélange fabriqué en (ii) se situe dans la plage allant de 10 à 75 % en poids.

21. Procédé selon l'une quelconque des revendications 16 à 20, dans lequel l'homogénéisation en (iii) a lieu par agitation, malaxage, secouage, vibration ou des combinaisons de deux ou plus d'entre eux.

22. Procédé selon l'une quelconque des revendications 16 à 21, dans lequel le revêtement en (iv) a lieu par revêtement par pulvérisation et/ou revêtement par lavage.

23. Procédé selon l'une quelconque des revendications 16 à 22, dans lequel l'étape (iv) est répétée une ou plusieurs fois.
